# EUROPEAN PATENT SPECIFICATION

(11) **EP 2 742 930 B1**
(45) Date of publication and mention of the grant of the patent: **10.10.2018**
(21) Application number: 12822436.7
(22) Date of filing: 07.08.2012
(51) Int. Cl.: A61K 8/49, A61K 8/31, A61Q 1/10, A61K 8/58, A61Q 7/00, A61K 8/06

(54) **COSMETIC EMULSION FOR EYEBROWS OR EYELASHES**
KOSMETISCHE EMULSION FÜR DIE WIMPERN ODER DIE AUGENBRAUEN
ÉMULSION COSMÉTIQUE POUR SOURCILS OU CILS

(30) Priority: 09.08.2011 JP 2011174084
(43) Date of publication of application: 18.06.2014
(73) Proprietor: Kao Corporation, Chuo-Ku Tokyo 103-8210 (JP)
(72) Inventor: KAIDA Kenji, Tokyo 131-8501 (JP); IMAI Takamitsu, Tokyo 131-8501 (JP)
(74) Representative: Hoffmann Eitle
(86) International application number: PCT/JP2012/070114
(87) International publication number: WO 2013/022003

(56) References cited:
- EP-A1- 0 743 311
- EP-A1- 2 236 128
- FR-A1- 2 873 024
- JP-A- 8 157 334
- JP-A- 10 152 486
- JP-A- 10 158 259
- JP-A- 11 035 575
- JP-A- 11 035 576
- JP-A- 2000 198 779
- JP-A- 2004 107 235
- JP-A- 2006 290 767
- JP-A- 2006 306 854
- JP-A- 2009 149 525
- JP-A- 2009 191 007
- US-A1- 2005 271 611
- US-A1- 2007 212 315
- Cosmetic Ingredient Review Panel Expert: "IsoParaffins", , 1 January 2010 (2010-01-01), pages 1-68, XP55227694, Retrieved from the Internet: URL:http://www.cir-safety.org/sites/defaul t/files/117_final_isopar.pdf [retrieved on 2015-11-11]

## Description

### Technical Field

The present invention relates to an emulsified cosmetic for eyebrows or eyelashes.

### Background Art

Eyebrows and eyelashes are objects to which makeup is to be applied. Therefore, to achieve more natural makeup, there is a demand to grow individual eyebrows and eyelashes thick. In addition, there is a demand to increase the number of existing eyebrows and eyelashes by suppressing shedding and falling out of the eyebrows and eyelashes.

One cosmetic for eyelashes proposed to satisfy these demands (Patent Literature 1) contains a uniform mixture of: a water-based high-viscosity base material thickened with a water-soluble polymer; and a solution prepared by dissolving, in ethanol, effective ingredients such as KANKOSO-301 having a hair matrix cell activating effect, a peripheral circulation improving effect, and a hair growth promoting effect, pantothenyl ethyl ether having a cell activating effect and a hair matrix cell activating effect, and a ginseng extract having a moisturizing and astringent effect.

It is contemplated that a hair growth and restoration agent containing a flavanonol derivative having a growth and restoration effect on head hair that is similar to eyebrows or eyelashes in that they grow from hair matrix cells (Patent Literature 2) is used as a growth and rastoration agent for eyebrows or eyelashe.

FR 2 873 024 A1 relates to a cosmetic treatment for preventing and/or decreasing hair fall or bristle fall, to favor the growth of hair and/or bristle, to increase the capillary density and/or to reduce heterogeneity of the diameter of the hair in a healthy individual, comprising applying a transdermal delivery system comprising a composition (which comprises at least one non-essential micronutrient (I)) to a zone of skin/scalp.

EP 0 743 311 A1 relates to flavanonol derivatives represented by formula (1): 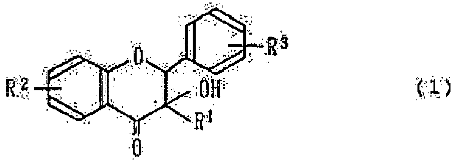 wherein R¹ represents an alkyl group, and each of R² and R³ represents a hydrogen atom, an alkyl group which may have a substituent, an alkoxyl group which may have a substituent, a hydroxyl group, a cyano group, or a halogen atom, as well as to hair-nourishing and hair-growing compositions containing the derivatives.

EP 2 236 128 A1 relates to a cosmetic for eyelashes which comprises (a) a silicone resin powder of particles having such a spiky-surface-sugar-candy-ball-like shape that small protrusions are present over the whole surface of each particle, (b) a volatile oil (e.g., low-boiling-point isoparaffinic hydrocarbon oil, low-boiling-point silicone oil, etc.), and (c) an oily gelling agent. Preferably, the cosmetic contains 1 - 20% by weight of component (a), 10 - 80% by mass of component (b) and 1 - 25% by mass of component (c). The cosmetic may optionally contain (d) a film-forming agent.

US 2005/271611 A1 relates to a cosmetic comprising a copolymer comprising specific acrylic acid monomer (A), specific polyoxyalkylene monomer (B) and specific organopolysiloxane monomer (C) as constituting monomers, wherein the content of monomer (A) is 20% by mass or more relative to the total amount of the constituting monomers.

US 2007/212315 A1 relates to compositions for eyelashes such as mascaras, topcoats and basecoats containing (a) at least one fatty alkoxylated dimeric compound; and (b) at least one polymeric compound selected from the group consisting of a polyorganosiloxane-containing polymer, a non-silicone polyamide copolymer, a hydrocarbon resin, a styrene-containing copolymer, and mixtures thereof.

Cosmetic ingredient Review Expert Panel Meeting, December 13-14, 2010, pages 1-68, relates to the ocular and skin irritation of C7-C40-isoparaffins, including isododecane.

### Citation List

### Patent Literature

Patent Literature 1: JP2002-128641A
Patent Literature 2: JP H8-157334A

### Summary of Invention

### Technical Problem

However, the cosmetic for eyelashes in Patent Literature 1 has a problem in that the results of follow-up experiments showed that the growth and restoration effect on eyelashes was not sufficient. In this cosmetic, ethanol is used as a resolvent for the effective ingredients such as KANKOSO-301, pantothenyl ethyl ether, and ginseng extract. Although the ability of ethanol to dissolve these effective ingredients is high, it is irritating to the eyes (more specifically, to the eyeballs, upper and lower eyelids with a thin epidermis, and mucous membranes on the back of the eyelids). Therefore, another problem is that, even when the amount of ethanol contained in the cosmetic for eyelashes is about the level used in Examples in Patent Literature 1, a certain number of users feel uncomfortable irritation. The water amount of this cosmetic is very high, i.e., about 90%. Therefore, the cosmetic also has a problem in that it is difficult to allow the cosmetic to contain water-insoluble effective ingredients dissolved therein.

The hair growth and restoration agent in Patent Literature 2 is used for sensitive areas around eyes. Therefore, the hair growth and restoration agent is required not to have a sticky feel, to resist adhesion to the eyeballs and mucous membranes around the eyeballs, and not to hinder the application of makeup such as eyebrow pencils, eyeliner, and eye shadow. In consideration of the above, the hair growth and restoration agent is required to be used not in a less volatile form such as hair cream, hair rinse, or hair shampoo but in a form in which the flavanonol derivative is dissolved in a volatile solvent. In Patent Literature 2, a large amount of ethanol is used as such a volatile solvent, from the viewpoint of providing pleasant stimulation to the scalp and of rapid drying properties. Therefore, it is feared that a problem of irritation due to ethanol may occur in the eyeballs, upper and lower eyelids, and mucous membranes of the eyelids.

In many women who use mascara regularly, the skin in areas around the eyes including areas around the roots of the eyebrows and eyelashes is dry and easily roughened. Therefore, regular application of a hair growth and restoration agent containing a large amount of alcohol, such as the hair growth and restoration agent described in Patent Literature 2 and containing the specific hair growing component, to the areas around the eyes causes rough skin such as tough dry skin. Accordingly, there is a demand for a cosmetic for eyebrows and eyelashes that is less irritating to the skin.

The present inventors have found a problem in that, when a growth and restoration agent for head hair that is expected to have a growth and restoration effect on eyebrows and eyelashes is actually applied to the eyebrows and eyelashes, the eyes are irritated, and the skin around the roots of the eyebrows and eyelashes is dry. In addition, elasticity of the skin is lost, and the skin becomes tough and dry.

### Solution to Problem

The present inventors have found the following. A specific flavanonol derivative known to have a growth and restoration effect on head hair is dissolved in a specific volatile oil used in place of ethanol to prepare an emulsified product. The obtained emulsified product (i.e., an emulsified cosmetic) acts directly on hair matrix cells, so that the eyebrows or eyelashes are thickened and more resistant to falling off and have a longer shedding period. A favorable growth and restoration effect that allows the eyebrows or eyelashes to grow longer can thereby be obtained. In addition to the above effect, irritation to the eyes can be reduced, dryness of the skin around the roots of the eyebrows and eyelashes can be suppressed, and the elasticity of the skin can be retained.

Accordingly, the present invention provides an emulsified cosmetic for eyebrows or eyelashes, the emulsified cosmetic containing: 0.05 % to 0.2 % by mass of a flavanonol derivative represented by the following formula (1); and 5 to 65% by mass of a volatile oil selected from volatile hydrocarbon oils and volatile silicone oils, wherein a mass ratio of the volatile oil to the flavanonol derivative being (parts by mass of the volatile oil)/(parts by mass of the flavanonol derivative) is 50 to 700.

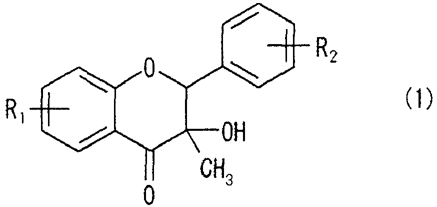

In the formula (1), R₁ and R₂ are each independently a hydrogen atom or a methyl group.

The present invention also provides a packaged emulsified cosmetic product for eyebrows or eyelashes that is used to grow and restore eyebrows or eyelashes, the packaged emulsified cosmetic product comprising: the above-described emulsified cosmetic for eyebrows or eyelashes; a container that contains the emulsified cosmetic for eyebrows or eyelashes; a lid capable of sealing the container; and a liquid applicator including a support shaft that is attached to the lid so as to be inserted into the container and an application portion attached to an end of the support shaft.

The present invention also provides, as a specific mode of the emulsified cosmetic, an emulsified growth and restoration agent for eyebrows or eyelashes, comprising: 0.05 to 0.2% by mass of a flavanonol derivative represented by the above-described formula (1); 5 to 65% by mass of a volatile oil selected from volatile hydrocarbon oils and volatile silicone oils; and water, wherein the flavanonol derivative is dissolved in the volatile oil in an oil phase, wherein a mass ratio of the volatile oil to the flavanonol derivative being (parts by mass of the volatile oil)/(parts by mass of the flavanonol derivative) is 50 to 700.

In addition, the present invention provides a method of use of the above-described emulsified cosmetic for eyebrows or eyelashes, comprising
applying the emulsified cosmetic for eyebrows or eyelashes to skin around roots of the eyebrows or the eyelashes once a day in order to promote growth and restoration of the eyebrows or the eyelashes. Advantageous Effects of Invention

The emulsified cosmetic for eyebrows or eyelashes of the present invention has a favorable growth and restoration effect on eyebrows or eyelashes. With the emulsified cosmetic, irritation to the eyes is suppressed, dryness of the skin around the roots of the eyebrows and the eyelashes is suppressed, and elasticity of the skin is not impaired.

### Brief Description of Drawings

FIG. 1 is a partially exploded front view illustrating one embodiment of a liquid application device including a liquid applicator of the present invention.
FIG. 2(a) is a perspective view of an application portion of the liquid applicator as viewed from the side on which a first surface of the application portion appears, and FIG. 2(b) is a perspective view of the application portion as viewed from the side on which its second surface appears.
FIG. 3(a) is a front view of the application portion of the liquid applicator, FIG. 3(b) is a side view of the application portion, FIG. 3(c) is a cross-sectional view taken along line c-c in FIG. 3(b), FIG. 3(d) is a cross-sectional view taken along line d-d in FIG. 3(b), and FIG. 3(e) is a cross-sectional view taken along line e-e in FIG. 3(b).
FIG. 4 is a front view of the application portion of the liquid applicator (a diagram corresponding to FIG. 3(a)).
FIG. 5 is a side view of the application portion of the liquid applicator (a diagram corresponding to FIG. 3(b)).
FIGs. 6(a) and 6(b) are diagrams sequentially illustrating the manner of pulling the applicator of the liquid application device out of a container.
FIG. 7 is a diagram illustrating an example of the use of the liquid application device including the liquid applicator of the present invention.
FIG. 8 is a diagram illustrating a state in which the application portion rotates pivotally about a smallest-width section of the application portion of the liquid applicator as a pivot axis.
FIG. 9 is a partial side view of a screw-like end portion of a screw-like metal applicator generally used for mascara.
FIG. 10A is a photograph of the eyelashes of the right eye which was not subjected to a hair growth effect test using an emulsified cosmetic in Example 1.
FIG. 10B is a photograph of the eyelashes of the left eye which was subjected to the hair growth effect test using the emulsified cosmetic in Example 1. Description of Embodiments

The emulsified cosmetic for eyebrows or eyelashes of the present invention contains a flavanonol derivative of the following formula (1) and further contains a volatile oil selected from volatile hydrocarbon oils and volatile silicone oils. The flavanonol derivative is dissolved in the volatile oil, and the solution forms an oil phase of the emulsion system.

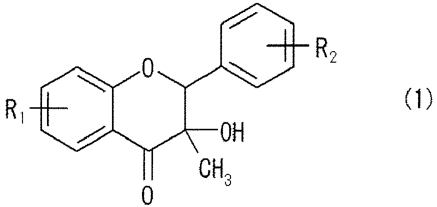

In the formula (1), R₁ and R₂ are each independently a hydrogen atom or a methyl group.

The flavanonol derivative of the formula (1) is preferably, for example, trans-3,4'-dimethylflavanonol described in Synthesis Example 1 in Japanese Patent No. 3131105 or trans-3,7-dimethylflavanonol described in Synthesis Example 18 in Japanese Patent No. 3131105 because of their ease of synthesis.

The flavanonol derivative of the formula (1) may be a single compound or a combination of two or more compounds.

The amount of the flavanonol derivative of the formula (1) contained in the emulsified cosmetic is 0.05% by mass or more, preferably 0.08% by mass or more, from the viewpoint of providing a favorable growth and restoration effect on eyebrows or eyelashes and a favorable bounce and resilience-imparting effect on the eyelashes. To ensure favorable solubility in the at least one volatile oil selected from volatile hydrocarbon oils and volatile silicone oils, the contained amount of the flavanonol derivative of the formula (1) is 0.2% by mass or less, preferably 0.12% by mass or less. More specifically, the amount of the flavanonol derivative of the formula (1) contained in the emulsified cosmetic is 0.05 to 0.2% by mass, preferably 0.08 to 0.12% by mass, in order to simultaneously achieve favorable solubility of the flavanonol derivative, a favorable growth and restoration effect of the emulsified cosmetic on the eyebrows or eyelashes, and a favorable bounce and resilience-imparting effect on the eyelashes.

The emulsified cosmetic of the present invention contains the volatile oil in order to dissolve the flavanonol derivative of the formula (1) to form an oil phase of the emulsion system. The reason that the volatile oil is used is as follows. When an emulsified cosmetic for eyebrows or eyelashes containing a nonvolatile oil instead of the volatile oil is applied to the roots of the eyebrows or eyelashes, the emulsified cosmetic causes a sticky feel and is more likely to adhere to the eyeballs and mucous membranes around the eyeballs, and this is likely to hinder makeup application. The volatile oil has a flash point of preferably 35 to 87°C. Examples of such a volatile oil may include volatile hydrocarbon oils and volatile silicone oils. From the viewpoint of the ability to dissolve a larger amount of the flavanonol derivative of the formula (1), volatile hydrocarbon oils can be preferably used.

The volatile hydrocarbon oil used may be any of those generally used for cosmetics. Examples of such a volatile hydrocarbon oil may include paraffinic hydrocarbon oils such as n-decane, n-undecane, and n-dodecane, isoparaffinic hydrocarbon oils such as isodecane and isododecane, and cycloparaffinic hydrocarbon oils such as cyclodecane and cyclododecane. Of these, isoparaffinic hydrocarbon oils having 8 to 18 carbon atoms are preferred, and isododecane is particularly preferred.

The volatile silicone oil used may be any of those generally used for cosmetics. Specific examples of such a volatile silicone oil may include: chain dimethylsiloxanes such as decamethyltetrasiloxane and octamethyltrisiloxane; and cyclic dimethylsiloxanes such as decamethylcyclopentasiloxane and octamethylcyclotetrasiloxane.

Examples of a commercial product of the volatile oil may include: MARUKASOL R (Maruzen Petrochemical Co., Ltd.); IP SOLVENT 1620, IP SOLVENT 2028, etc. (Idemitsu Kosan Co., Ltd.); and KF-995, KF-994, KF-96L-1.5CS, KF-96A-1CS, etc., (Shin-Etsu Chemical Co., Ltd.).

The amount of the volatile oil contained in the emulsified cosmetic is 5% by mass or more, preferably 10% by mass or more, more preferably 15% by mass or more, and still more preferably 20% by mass or more, in order to dissolve the flavanonol derivative of the formula (1). The contained amount of the volatile oil is 65% by mass or less, preferably 60% by mass or less, more preferably 55% by mass or less, and still more preferably 45% by mass or less, in order to reduce irritation when the emulsified cosmetic is applied to the roots of the eyebrows or eyelashes. More specifically, the amount of the volatile oil contained in the emulsified cosmetic is 5 to 65% by mass, preferably 10 to 60% by mass, more preferably 15 to 55% by mass, and still more preferably 20 to 45% by mass, in order to simultaneously achieve favorable solubility of the flavanonol derivative and a reduction in irritation caused by the emulsified cosmetic.

If the mass ratio of the volatile oil to the flavanonol derivative, i.e., the mass ratio (the parts by mass of the volatile oil)/(the parts by mass of the flavanonol derivative), is too small or too large, dryness of the skin around the roots of the eyebrows and eyelashes cannot be suppressed, and the elasticity of the skin cannot be retained. Therefore, the mass ratio is 50 or more, preferably 100 or more, more preferably 200 or more and is 700 or less, preferably 600 or less, and more preferably 450 or less. More specifically, the mass ratio (the parts by mass of the volatile oil)/(the parts by mass of the flavanonol derivative) is 50 to 700, preferably 100 to 600, and more preferably 200 to 450.

The oil phase containing the flavanonol derivative of the formula (1) may contain an oil-soluble or oil-dispersible thickener and an oil-soluble or oil-dispersible gelling agent, in order to, for example, adjust the viscosity of the cosmetic. Examples of the oil-soluble gelling agent may include dextrin palmitate. Examples of the oil-dispersible gelling agent may include wax, colloidal silica, and organic-modified clay minerals. Of these, organic-modified clay minerals can be preferably used from the viewpoint of reducing the stickiness of the emulsified cosmetic and its ease of handling during production and particularly because a heating step when the volatile oil is used is unnecessary. Preferred examples of such an organic-modified clay mineral may include a product obtained by treating a laminar clay mineral such as bentonite, magnesium aluminum silicate, Laponite, or hectorite with a quaternary ammonium salt-type cationic surfactant to substitute interlayer sodium ions in the laminar clay mineral with quaternary ammonium ions. Specific examples may include dimethyl alkyl ammonium hectorite, benzyl dimethyl stearyl ammonium hectorite, and magnesium ammonium silicate treated with distearyl dimethyl ammonium chloride. Examples of a commercial product of the organic-modified clay mineral may include BENTONE 38, BENTONE 27, and BENTONE 38VCG (Elementis Japan). Preferably, from the viewpoint of improving workability, the organic-modified clay mineral used may be a pre-mixed gel obtained by pre-dispersing the organic-modified clay mineral in a solvent. Examples of a commercial product of the pre-mixed gel may include BENTONE GEL ISDV (Elementis Japan).

To prevent excessive running of the emulsified cosmetic, the amount of the organic-modified clay mineral contained in the emulsified cosmetic is preferably 0.1% by mass or more, more preferably 0.2% by mass or more, still more preferably 0.3% by mass or more, and yet more preferably 0.5% by mass or more. To maintain good applicability, the contained amount of the organic-modified clay mineral is preferably 10% by mass or less, more preferably 7% by mass or less, still more preferably 5% by mass or less, and yet more preferably 4% by mass or less. More specifically, the amount of the organic-modified clay mineral contained in the emulsified cosmetic is preferably 0.1 to 10% by mass, more preferably 0.2 to 7% by mass, still more preferably 0.3 to 5% by mass, and yet more preferably 0.5 to 4% by mass, in order to simultaneously impart favorable resistance to running and favorable applicability to the emulsified cosmetic.

The emulsion system of the emulsified cosmetic for eyebrows and eyelashes of the present invention can be selected from the W/O type, the O/W type, the O/W/O type, and the W/O/W type. From the viewpoint of suppressing irritation to the skin, which is one indicator of selection, an emulsion system in which the continuous phase is a water phase is preferred. From the viewpoint of allowing the flavanonol derivative to penetrate deep into the roots of hair, a system in which the continuous phase is the oil phase in which the flavanonol derivative is dissolved is preferred. To satisfy these viewpoints simultaneously in a well-balanced manner, the emulsion system is preferably of the W/O type with relatively high water amount. The contained amount of the water phase is preferably 35% by mass or more, more preferably 40% by mass or more, and still more preferably 45% by mass or more and is preferably 95% by mass or less, more preferably 85% by mass or less, and still more preferably 75% by mass or less. More specifically, the contained amount of the water phase is preferably 35 to 95% by mass, more preferably 40 to 85% by mass, and still more preferably 45 to 75% by mass.

A stable emulsion system can be formed for any formulation when the ratio of water in the water phase in the emulsified cosmetic for eyebrows and eyelashes of the present invention is 60% by mass or more.

The emulsified cosmetic for eyebrows and eyelashes of the present invention may further contain an emulsifier to stabilize the emulsified state of the emulsified cosmetic. An emulsifier appropriately selected according to the intended emulsion system may be used.

In the emulsified cosmetic for eyebrows and eyelashes of the present invention, the emulsifier that may be added to the oil phase during production of the cosmetic is preferably a silicone-based emulsifier, from the viewpoint of forming a stable W/O type emulsion system and reducing the sticky feel of the emulsified product. A silicone-based emulsifier having an HLB value of 3 to 10 is preferred, and a silicone-based emulsifier having an HLB value of 4 to 8 is more preferred. Among the silicone-based emulsifiers, polyoxyethylene methyl polysiloxane etc. is preferred. Examples of a commercial product of the silicone-based emulsifier may include: PEG-12 dimethicone, for example, SH3775M (Dow Corning Toray Co., Ltd.); PEG-9 polydimethylsiloxyethyl dimethicone, for example, KF-6028 (Shin-Etsu Chemical Co., Ltd.); and PEG-3 dimethicone, for example, KF-6015 (Shin-Etsu Chemical Co., Ltd.). More specifically, PEG-12 dimethicone, PEG-9 polydimethylsiloxyethyl dimethicone, and PEG-3 dimethicone are preferred.

The amount of the silicone-based emulsifier contained in the emulsified cosmetic for eyebrows or eyelashes is preferably 0.1% by mass or more, more preferably 0.5% by mass or more, and still more preferably 1% by mass or more, from the viewpoint of forming a stable emulsion system. Particularly, from the viewpoint that the sticky feel can be reduced, the contained amount of the silicone-based emulsifier is preferably 5% by mass or less, more preferably 4% by mass or less, and still more preferably 3% by mass or less. More specifically, to achieve emulsification stability and less stickiness simultaneously, the contained amount of the silicone-based emulsifier is preferably 0.1 to 5% by mass, more preferably 0.5 to 4% by mass, and still more preferably 1 to 3% by mass.

In the emulsified cosmetic for eyebrows and eyelashes of the present invention, the emulsifier that may be added to the water phase during production is preferably a nonionic surfactant from the viewpoint of forming a stable O/W type emulsion system and reducing the sticky feel of the emulsified product. The emulsifier is more preferably a nonionic surfactant having an HLB value of 3 to 17 and still more preferably a combination of a nonionic surfactant having an HLB value of 3 to 7 and a nonionic surfactant having an HLB value of 13 to 17. Examples of such a nonionic surfactant may include fatty acid ester-based nonionic surfactants such as: sorbitan stearate, for example, RHEODOL SP-S10V (Kao Corporation); and polysorbate 60, for example, RHEODOL TW-S120V (Kao Corporation).

The amount of such a nonionic surfactant contained in the emulsified cosmetic for eyebrows or eyelashes is preferably 0.1% by mass or more, more preferably 0.3% by mass or more, and still more preferably 0.6% by mass or more, from the viewpoint of stabilizing the emulsified state. Particularly, from the viewpoint that the sticky feel can be reduced, the contained amount of the nonionic surfactant is preferably 5% by mass or less, more preferably 4% by mass or less, and still more preferably 3% by mass or less. More specifically, to achieve emulsification stability and suppression of stickiness after application simultaneously, the contained amount of the nonionic surfactant is preferably 0.1 to 5% by mass, more preferably 0.3 to 4% by mass, and still more preferably 0.6 to 3% by mass.

The emulsified cosmetic for eyebrows and eyelashes of the present invention may contain components used for general cosmetics so long as the effects of the present invention are not impaired. Examples of such components may include additives such as a surfactant other than the above surfactants, a coating agent, a fatty acid, an alcohol such as a higher alcohol and a polyhydric alcohol, a nonvolatile ester oil, a nonvolatile hydrocarbon oil such as liquid isoparaffin, a nonvolatile silicone oil, a fluorine oil, a humectant, a pH adjuster, a neutralizer, an inorganic salt, an ultraviolet absorber, an ultraviolet scattering agent, a humectant, a perfume, an antioxidant, a preservative such as phenoxyethanol, a sequestering agent, an antifoaming agent, a dye, a pigment, and various extracts.

To prevent running of the emulsified cosmetic on the skin when the emulsified cosmetic is applied to the skin around the roots of the eyebrows or eyelashes and to suppress dryness of the skin around the roots, the viscosity of the emulsified cosmetic for eyebrows and eyelashes of the present invention at 25°C is preferably 1 Pa·s or more, more preferably 5 Pa·s or more, and still more preferably 10 Pa·s or more. From the viewpoint of preventing unevenness and stickiness, the viscosity of the emulsified cosmetic at 25°C is preferably 400 Pa·s or less, more preferably 300 Pa·s or less, and still more preferably 200 Pa·s or less. More specifically, from the viewpoint of suppressing running during application, dryness of the skin around the root, uneven application, and stickiness simultaneously, the viscosity of the emulsified cosmetic for eyebrows or eyelashes at 25°C is preferably 1 to 400 Pa·s, more preferably 5 to 300 Pa·s, and still more preferably 10 to 200 Pa·s.

The viscosity is measured using a B-type viscometer (TVB-10M, Toki Sangyo Co., Ltd.) under the condition of 25°C with a rotor M3 rotating at a rotation speed of 1.5 r/min when the viscosity is 80 Pa·s or less. When the viscosity exceeds 80 Pa·s, the measurement is performed with the rotor M3 rotating at a rotation speed of 0.3 r/min.

With attention paid to the effects of the above-described emulsified cosmetic for eyebrows and eyelashes of the present invention, the emulsified cosmetic can preferably take a form of "an emulsified growth and restoration agent for eyebrows or eyelashes that contains: 0.05 % to 0.2 % by mass of the flavanonol derivative represented by the formula (1) above; 5 to 65% by mass of the volatile oil selected from volatile hydrocarbon oils and volatile silicone oils and used to dissolve the flavanonol derivative; and water; wherein the flavanonol derivative is dissolved in the volatile oil."

The emulsified cosmetic for eyebrows and eyelashes of the present invention can be produced by any known method of producing an emulsified cosmetic. For example, the flavanonol derivative of the formula (1) is dissolved in the volatile oil, and, if necessary, an organic-modified clay mineral is mixed therewith to prepare an oil phase. Then the oil phase is mixed with a water phase prepared separately and emulsified therein, whereby the emulsified cosmetic can be prepared. The "emulsified growth and restoration agent for eyebrows or eyelashes," which is a specific mode of the present invention, can be produced in a similar manner.

To achieve the growth and restoration effect on eyebrows or eyelashes and the bounce and resilience-imparting effect on eyelashes, it is preferable that the emulsified cosmetic for eyebrows and eyelashes of the present invention be applied to the skin around the roots of the eyebrows or eyelashes generally once a day. The amount of the cosmetic per application for one eye is preferably 0.002 g or more, more preferably 0.003 g or more, and still more preferably 0.005 g or more and is preferably 0.04 g or less, more preferably 0.03 g or less, and still more preferably 0.025 g or less. More specifically, the amount per application for one eye is preferably 0.002 to 0.04 g, more preferably 0.003 to 0.03 g, and still more preferably 0.005 to 0.025 g. When the cosmetic is used continuously for one month, its effects can be actually realized, although there are variations among individuals.

Preferably, the above-described emulsified cosmetic of the present invention is charged into a container and used in the form of a "packaged emulsified cosmetic product" for application to eyebrows or eyelashes. The "packaged emulsified cosmetic product" includes the emulsified cosmetic of the present invention, the container containing the emulsified cosmetic, a lid that can seal the container, and a liquid applicator including a support shaft that is attached to the lid so as to be inserted into the container and an application portion attached to an end of the support shaft.

No particular limitation is imposed on the liquid applicator so long as a proper amount of the emulsified cosmetic can be applied. From the viewpoint of applying the emulsified cosmetic to fine areas and preventing irritation to the applied area, the liquid applicator is preferably a brush, a resin-molded applicator, a metal-molded applicator, an applicator produced by planting bristles on one of the above applicators, etc. The liquid applicator is also preferably a manual or electric vibrating or rotating applicator because it can apply the cosmetic more uniformly. During the process of application using these applicators, the vicinity of the roots of eyebrows or eyelashes is massaged with the application portion, so that the penetration of the effective ingredient is facilitated. A preferred specific configuration of the liquid applicator will next be described.

The liquid applicator preferably used in the present invention includes a support shaft and an application portion connected to an end of the support shaft and having a shape elongated in the extending direction of the support shaft. The application portion is elastically deformable when an external force is applied thereto during the operation for applying the liquid. The axis of the application portion in its extending direction is inclined so as to cross the axis of the support shaft. The application portion has a first surface having a substantially flat or slightly convex shape and a second surface opposite to the first surface. The application portion further has a smallest-width section when the application portion is viewed from the side on which the first surface appears. The width of the application portion gradually increases from the smallest-width section toward an end and then gradually decreases toward the end. When the application portion is viewed in a direction orthogonal to a plane passing through the axis of the support shaft and the axis of the application portion, the application portion has a smallest-thickness section. The application portion further has a largest-thickness section formed as a protrusion of the second surface between the smallest-thickness section and the end of the application portion. The thickness of the application portion gradually decreases from the largest-thickness section toward the end of the application portion.

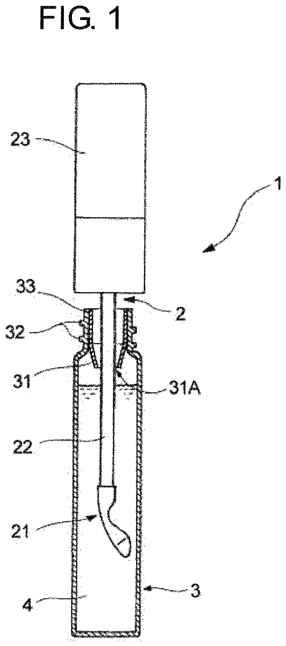

FIG. 1 is a partially exploded front view of one embodiment of the "packaged emulsified cosmetic product" 1 for eyebrows or eyelashes that is provided with the above liquid applicator. The "packaged emulsified cosmetic product" 1 includes a liquid applicator 2 for application of the emulsified cosmetic 4 for eyebrows or eyelashes and a container 3 for containing the emulsified cosmetic 4 for eyebrows or eyelashes. Each of the liquid applicator 2 and the container 3 will next be described.

The container 3 that can contain the emulsified cosmetic 4 for eyebrows or eyelashes has an elongated closed-end tubular shape and includes an opening 33 that opens upward. A threaded portion 32 is provided on the outer circumferential surface of the opening 33. The threaded portion 32 can be screwed with a threaded portion (not shown) provided on the inner circumferential surface of a lid 23, described later, of the liquid applicator 2.

A scraping valve 31 for properly scraping off the excess amount of the emulsified cosmetic 4 for eyebrows or eyelashes adhering to the application portion 21 and support shaft 22 of the liquid applicator 2 is provided near the opening 33. The scraping valve 31 has a funnel shape reduced in diameter from the opening 33 of the container 3 toward its bottom and includes a scraping opening 31A at the lower end of the funnel shape. The scraping opening 31A is configured such that the application portion 21 and support shaft 22 of the liquid applicator 2 described later can pass therethrough. In a cross-section orthogonal to the lengthwise direction of the container 3, the scraping opening 31A is generally formed into a circular shape at a substantially central portion of the scraping valve 31, but this is not a limitation. The diameter of the scraping opening 31A is configured such that the application portion 21 located at the end of the liquid applicator 2 can be inserted into the container 3 and pulled out of the container 3.

The above scraping valve 31 is formed from an elastically deformable material such as rubber. In FIG. 1, the scraping valve 31 is located at a position slightly spaced downward from the opening 33. Instead, the scraping valve 31 may be disposed at the position of the opening 33. The vicinity of the opening 33 of the container body 3 includes a section extending from a portion slightly spaced apart from the opening 33 to the opening 33.

The liquid applicator 2 used in combination with the container 3 includes the application portion 21 and the support shaft 22, as described above. The application portion 21 and the support shaft 22 may be integrally formed of a single material. Alternatively, the application portion 21 and the support shaft 22 may be two separate components produced in advance, and these components may be joined by prescribed means. In either case, the application portion 21 can be elastically deformed by external force applied thereto during the operation for applying the emulsified cosmetic 4 for eyebrows or eyelashes. For this purpose, any of various elastomers such as natural rubber, butadiene rubber, isoprene rubber, nitrile rubber, chloroprene rubber, silicone resin, and polyurethane can be used as the material of the application portion 21. Particularly, the use of a thermoplastic elastomer is preferred because the application portion 21 can be produced by injection molding.

When the application portion 21 and the support shaft 22 are produced separately and then joined, it is preferable to fit them with each other to join them. Other examples of the joining method may include a method in which the application portion 21 and the support shaft 22 are secured to each other by plastically deforming one of them by, for example, punching. A known technique such as bonding using an adhesive may be appropriately used.

The liquid applicator 2 is attached to the lid 23 through one end of the support shaft 22, as described above. The lid 23 has the threaded portion (not shown) formed on its inner circumferential surface, as described above. This threaded portion can be screwed with the threaded portion 32 of the container 3 described above. When the lid 23 is screwed with the threaded portion 32 of the container 3, the "packaged emulsified cosmetic product" 1 has a substantially columnar shape. In this state, the application portion 21 is located slightly above the bottom of the container 3.

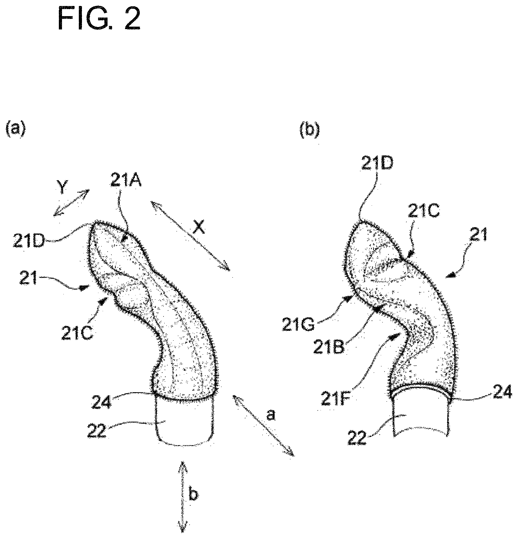

FIGs. 2(a) and 2(b) show enlarged views of the application portion 21. It should be noted that the vertical orientation of the application portion 21 in FIG. 1 is reversed in FIGs. 2(a) and 2(b). FIG. 2(a) is a perspective view of the application portion 21 as viewed from the side on which the first surface 21A appears, and FIG. 2(b) is a perspective view of the application portion 21 as viewed from the side on which the second surface 21B appears. The application portion 21 has a shape elongated in the extending direction of the support shaft 22. More specifically, the application portion 21 has a vertically elongated shape with a lengthwise direction X being in the extending direction of the support shaft 22 and a width direction Y being in a direction orthogonal to the lengthwise direction X.

The application portion 21 has the first surface 21A and the second surface 21B opposite thereto. The first surface 21A is a substantially flat surface or a slightly convex surface. The convex surface used herein means a convex surface extending in the lengthwise direction X of the application portion 21 and/or extending in the width direction Y of the application portion 21. The second surface 21B is an undulating surface with undulations.

The application portion 21 is inclined at a prescribed angle with respect to the support shaft 22. More specifically, as shown in FIG. 2(a), an axis "a" of the application portion 21 in its extending direction is inclined so as to cross an axis "b" of the support shaft 22 in its extending direction. With the liquid applicator 2 inserted into the container 3, the application portion 21 is inclined with respect to the axis of the support shaft 22 such that the first surface 21A of the application portion 21 faces toward the bottom of the container 3, as shown in FIG. 1. Since the application portion 21 and the support shaft 22 are joined with such an inclined relation therebetween, the emulsified cosmetic 4 for eyebrows or eyelashes can be applied easily using the liquid applicator 2.

Preferably, the surface of the application portion 21 has been subjected to bristle planting processing. Preferably, fibers used for the bristle planting processing have a length of 0.1 to 3 mm and a thickness of 0.5 to 5 dtex. Two or more different types of fibers within the above length and thickness ranges may be used in combination. The material of the fibers may be selected appropriately according to the type etc. of the emulsified cosmetic 4 for eyebrows or eyelashes. To subject the surface of the application portion 21 to bristle planting processing, a known method such as electrostatic flocking may be appropriately used.

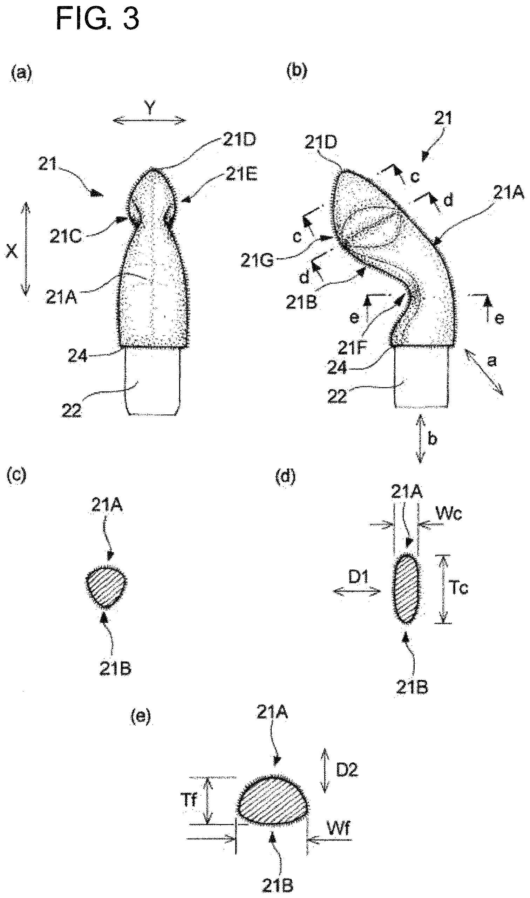

FIGs. 3(a) to 3(e) show a front view, a side view, and horizontal cross-sectional views of the application portion 21 shown in FIGs. 2(a) and 2(b). As described above, the surface of the application portion 21 has been subjected to bristle planting processing. However, the bristle planting processing may not be performed for some types of emulsified cosmetics 4 for eyebrows or eyelashes. Irrespective of whether or not the bristle planting processing is performed, it is preferable that the entire exposed surfaces of the application portion 21 before bristle planting processing be formed only from curved surfaces. In other words, it is preferable that the application portion 21 before bristle planting processing be smooth with no corners.

The exposed surfaces of the application portion 21 before bristle planting processing may be smooth. In other words, the application portion 21 before bristle planting processing may not have, on the exposed surfaces, various fine undulations such as wrinkle-, crimp-, and satin-like undulations. Instead of employing such a surface state, the exposed surfaces may have various fine undulations such as wrinkle-, crimp-, and satin-like undulations. Alternatively, the exposed surfaces may be porous. The surface state of the exposed surfaces may be selected and determined according to the type of the emulsified cosmetic 4 for eyebrows or eyelashes.

As shown in FIGs. 2 and 3, the application portion 21 has a smallest-width section 21C as viewed from the side on which the first surface 21A appears. The above width is a length in the Y direction in FIG. 3(a). The smallest-width section 21C is located forward of a position at which the application portion 21 is divided into two parts in its lengthwise direction. The smallest-width section 21C is located at a position spaced apart from a forward end 21D of the application portion 21. Only one smallest-width section 21C is illustrated. However, two or more smallest-width sections may be present, or a smallest-width section extending over a certain length may be present. The width of the application portion 21 gradually increases from the smallest-width section 21C toward the forward end 21D of the application portion 21. Then the width gradually decreases toward the forward end 21D through a large-width section 21E. As viewed from the side on which the first surface 21A appears, the application portion 21 is gradually increased in width from the smallest-width section 21C toward the rear end of the application portion 21. The width of the application portion 21 and the width of the support shaft 22 coincide substantially with each other at a joint portion 24 between the application portion 21 and the support shaft 22. The application portion 21 has no small-width portion in a section between the smallest-width section 21C and the joint portion 24. The application portion 21 illustrated in FIG. 2(a) is left-right symmetric. However, the application portion 21 is not necessarily left-right symmetric.

As shown in FIG. 3(d), in the smallest-width section 21C, its width Wc is smaller than the thickness Tc of the smallest-width section 21C. Since the width Wc is smaller than the thickness Tc and the application portion 21 is elastically deformable, the application portion 21 is bent at the smallest-width section 21C more easily in an in-plane direction of the first surface 21A (a direction denoted by numeral D1 in FIG. 3(d)) than in a direction orthogonal to the first surface.

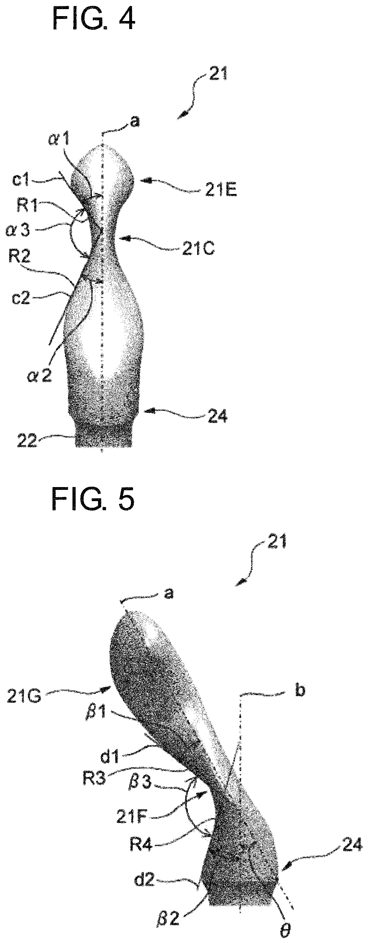

As shown in FIG. 4, the extent to which the application portion 21 is gradually increased in width from the smallest-width section 21C toward the large-width section 21E when viewed from the side on which the first surface 21A appears is preferably such that the angle α1 between straight line c1 and the axis "a" of the application portion 21 is within the range of 5° < α1 < 60°, and particularly 10° < α1 < 45°. By setting the angle α1 within this range, the application portion 21 is unlikely to be caught on the scraping valve 31 when the liquid applicator 2 is pulled out of the container 3, so that the liquid applicator 2 can be easily pulled out of the container 3. In addition, the vicinity of the smallest-width section 21C is effectively scraped, so that excess liquid is less likely to remain on the application portion 21.

From a similar reason, the angle α2 between straight line c2 and the axis "a" of the application portion. 21 is within the range of preferably 5° < α2 < 60° and more preferably 10° < α2 < 45°, as shown in FIG. 4. The values of the angles α1 and α2 may be the same or different. As shown in FIG. 4, the angle α3 between the lines c1 and c2 is preferably about 60° < α3 < 150°. By setting the angle α3 within this range, the vicinity of the smallest-width section 21C is effectively scraped. In addition, the liquid is less likely to remain on the smallest-width section 21C. Moreover, the application portion 21 is likely to be bent left and right in the figure with the smallest-width section 21C serving as a pivot axis.

The straight line c1 in FIG. 4 is a tangential line of a contour R1 of the application portion 21 at an intermediate position between the smallest-width section 21C and the large-width section 21E. The contour R1 is part of the contour of the application portion 21 that is projected onto a plane parallel to the first surface 21A of the application portion 21. The straight line c2 is a tangential line of a contour R2 of the application portion 21 at an intermediate position between the smallest-width section 21C and a largest-width section located at a position closer to the joint portion 24 than the smallest-width section 21C. The contour R2 is part of the above-described contour. If, for example, the contours R1 and R2 are not smooth (for example, are undulated or discontinuous) and the gradients of the tangential lines vary greatly depending on the points at which the tangential lines are drawn, curved profiles of the contours R1 and R2 produced using about 50% of the entire lengths of the contours R1 and R2 near their central sections are used, and straight lines obtained by approximation using the least square method etc. may be used as the straight lines c1 and c2. More specifically, such an approximate straight line is obtained by the least square method etc. using coordinate data of a group of points or functions for curves of about 25 to 75 sections out of 100 sections of the entire length. When the contours R1 and R2 have inflection points near the centers of the entire lengths, tangential lines passing through the inflection points may be used as the straight lines c1 and c2. In FIG. 4, the straight line c1 and the straight line c2 intersect each other on the axis "a," but the point of intersection between the straight lines c1 and c2 is not limited thereto.

Returning to FIGs. 2 and 3, the application portion 21 has a smallest-thickness section 21F when viewed in a direction orthogonal to a plane passing through the axis "b" of the support shaft 22 and the axis "a" of the application portion 21, specifically, a plane parallel to the sheet of FIG. 3(b), (i.e., a direction perpendicular to the sheet of FIG. 3(b)), as shown in these figures. The above thickness is the length of a line that is orthogonal to the axis "a" of the application portion 21 and crosses the application portion 21 when the application portion 21 is viewed in a direction orthogonal to the plane passing through the axis "b" of the support shaft 22 and the axis "a" of the application portion 21.

In the application portion 21, the second surface 21B protrudes between the smallest-thickness section 21F and the forward end 21D of the application portion 21 to form a substantially pyramidal protruding section. A largest-thickness section 21G of the application portion 21 is present at the apex of the substantially pyramidal protruding section. Only one largest-thickness section 21G is illustrated. However, two or more largest-thickness sections may be present, or a largest-thickness section extending over a certain length may be present. The thickness of the application portion 21 gradually decreases from the position of the largest-thickness section 21G toward the forward end 21D. The thickness of the application portion 21 gradually increases from the position of the smallest-thickness section 21F toward the position of the largest-thickness section 21G. The thickness of the application portion 21 gradually increases from the position of the smallest-thickness section 21F toward the rear end of the application portion 21. The outline of the application portion 21 and the outline of the support shaft 22 substantially coincide with each other at the joint portion 24 between the application portion 21 and the support shaft 22.

As shown in FIG. 3(e), in the smallest-thickness section 21F, the width Wf of the smallest-thickness section 21F is larger than the thickness Tf of the smallest-thickness section 21F. Since the width Wf is larger than the thickness Tf and the application portion 21 is elastically deformable, the application portion 21 can be bent at the smallest-thickness section 21F more easily in a direction orthogonal to the first surface 21A (a direction denoted by numeral D2 in FIG. 3(e)) than in the in-plane direction of the first surface.

As shown in FIGs. 2 and 3, in the application portion 21, the smallest-width section 21C is located closer to the forward end 21D of the application portion 21 than the smallest-thickness section 21F. However, the positional relation between the smallest-width section 21C and the smallest-thickness section 21F is not limited thereto. For example, the smallest-width section 21C may be closer to the rear end of the application portion 21 than the smallest-thickness section 21F, or the smallest-width section 21C and the smallest-thickness section 21F may be located at substantially the same position.

In the positional relation between the smallest-width section 21C and the largest-thickness section 21G in the embodiment shown in FIGs. 2 and 3, the smallest-width section 21C and the largest-thickness section 21G are located at substantially the same position. However, the positional relation between these sections is not limited thereto. For example, the smallest-width section 21C may be located closer to the forward end 21D of the application portion 21 than the largest-thickness section 21G, or the smallest-width section 21C may be closer to the rear end of the application portion 21 than the largest-thickness section 21G.

As shown in FIG. 3(a), when the application portion 21 is viewed from the side on which the first surface 21A appears, the forward end 21D of the application portion 21 is tapered and rounded. As shown in FIG. 3(b), when the application portion 21 is viewed in a direction orthogonal to the plane passing through the axis "b" of the support shaft 22 and the axis "a" of the application portion 21, the forward end 21D of the application portion 21 is tapered and rounded. As described above, the forward end 21D of the application portion 21 is rounded when viewed in any direction. In other words, although the forward end 21D has a tapered shape, it is not acute when viewed in any direction.

As shown in FIG. 5, the extent to which the application portion 21 is gradually increased in width from the smallest-thickness section 21F toward the largest-thickness section 21G when viewed in the direction orthogonal to the plane passing through the axis "b" of the support shaft 22 and the axis "a" of the application portion 21 is preferably such that the angle β1 between straight line d1 and the axis "a" of the application portion 21 is within the range of 5° < β1 < 60°, and particularly 10° < β1 < 45°. By setting β1 within this range, the application portion 21 is unlikely to be caught on the scraping valve 31 when the liquid applicator 2 is pulled out of the container 3, so that the liquid applicator 2 can be easily pulled out of the container 3. In addition, the vicinity of the smallest-width section 21C is effectively scraped, so that excess liquid is less likely to remain on the application portion 21.

From a similar reason, the angle β2 between straight line d2 and the axis "b" of the support shaft 22 is within the range of preferably 0° < β2 < 60° and still more preferably 5° < β2 < 45°, and the angle β3 between the straight lines d1 and d2 is preferably 60° < β3 < 150°, as shown in FIG. 5. The values of the angles β1 and β2 may be the same or different.

The straight line d1 in FIG. 5 is a tangential line of a contour R3 of the application portion 21 at an intermediate position between the smallest-thickness section 21F and the largest-thickness section 21G, when the application portion 21 is viewed in a direction orthogonal to a plane passing through the axis "b" of the support shaft 22 and the axis "a" of the application portion 21. More specifically, the contour R3 is part of the contour of the application portion 21 that is projected onto a plane parallel to the above plane. The straight line d2 is a tangential line of a contour R4 of the application portion 21 at an intermediate position between the smallest-thickness section 21F and a largest-thickness section located at a position closer to the joint portion 24 than the smallest-thickness section 21F. The contour R4 is part of the above-described contour. If, for example, the contours R3 and R4 are not smooth (for example, are undulated or discontinuous) and the gradients of the tangential lines vary greatly depending on the points at which the tangential lines are drawn, curved profiles of the contours R3 and R4 produced using about 50% of the entire lengths of the contours R3 and R4 near their central sections are used, and straight lines obtained by approximation using the least square method etc. may be used as the straight lines d1 and d2. More specifically, such an approximate straight line is obtained by the least square method etc. using coordinate data of a group of points or functions for curves of about 25 to 75 sections out of 100 sections of the entire length. When the contours R3 and R4 have inflection points near the centers of the entire lengths, tangential lines passing through the inflection points may be used as the straight lines d1 and d2.

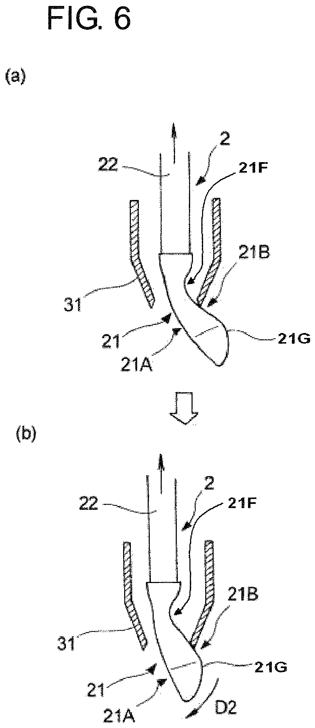

FIGs. 6(a) and 6(b) illustrate the manner of pulling the liquid applicator 2 in the "packaged emulsified cosmetic product" 1 having the above-described structure out of the container 3. In a state before use, the application portion 21 of the liquid applicator 2 is located slightly above the bottom of the container 3, as shown in FIG. 1. When the liquid applicator 2 in this state is pulled up, the second surface 21B of the application portion 21 comes into contact with the lower end of the scraping valve 31 as shown in FIG. 6(a) because the application portion 21 is inclined, and the excess emulsified cosmetic present on the second surface 21B is scraped off by the scraping valve 31. As the liquid applicator 2 is further pulled up, the protruding and swelling largest-thickness section 21G of the application portion 21 comes close to the lower end of the scraping valve 31 as shown in FIG. 6(b), so that the liquid applicator 2 is not easily pulled out.

As described above, the smallest-thickness section 21F can be bent more easily in the direction orthogonal to the first surface 21A of the application portion 21 (the direction denoted by numeral D2 in FIG. 6(b)) than in the in-plane direction of the first surface. Therefore, when the liquid applicator 2 in the state shown in FIG. 6(b) is further pulled up, the application portion 21 is bent in a direction indicated by an arrow with the position of the smallest-thickness section 21F serving as a pivot axis (i.e., is pivotally rotated), so that the abutment between the application portion 21 and the scraping valve 31 is relaxed. Therefore, the application portion 21 can be pulled out without the need of excessive force. In addition, since the swelling and protruding largest-thickness section 21G of the application portion 21 and its vicinity are effectively scraped by the scraping valve 31, the excess emulsified cosmetic adhering to the application portion 21 can be successfully scraped off. Therefore, dripping of the emulsified cosmetic from the application portion 21 during the operation for applying the emulsified cosmetic is effectively prevented.

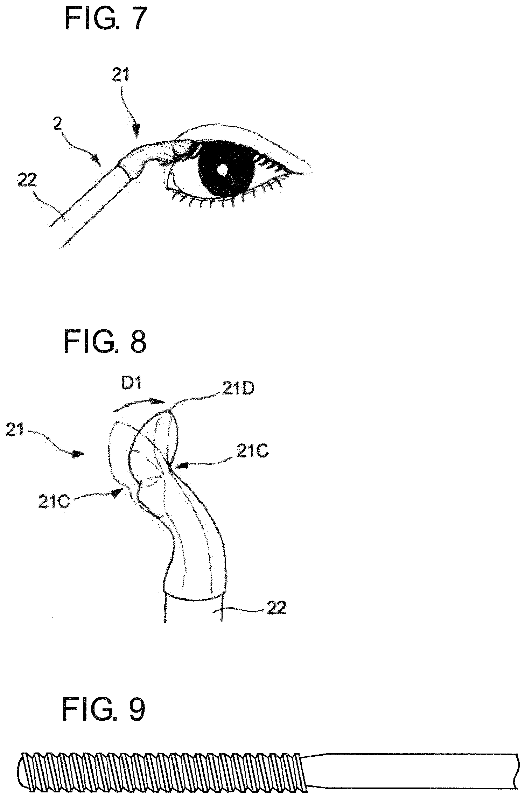

FIG. 7 illustrates an example of the use of the "packaged emulsified cosmetic product" 1 in this embodiment having the above-described structure. To apply the emulsified cosmetic for growing and restoring eyebrows or eyelashes to the roots of the eyelashes, first, the operation shown in FIGs. 6(a) and 6(b) above is performed to remove the excess emulsified cosmetic adhering to the application portion 21 so that the emulsified cosmetic is prevented from being dripped during the application operation. Next, as shown in FIG. 7, the forward end of the application portion 21 is brought into contact with the roots of eyelashes to apply the cosmetic solution to the root of the eyelashes. As described above, since the forward end 21D of the application portion 21 of the liquid applicator 2 has a tapered shape, the emulsified cosmetic can be applied to the application object even when it is fine. In addition, since the forward end 21D of the application portion 21 is not sharp but is rounded, the forward end 21D can be brought close to the eye of the user with a sense of safety.

As described above, the axis "a" of the application portion 21 in its extending direction is inclined so as to cross the axis "b" of the support shaft 22. One advantage of the inclined application portion 21 is that, when the application portion 21 is used to apply the emulsified cosmetic with the first surface 21A facing upward and the second surface 21B facing downward, the field of view of the user is ensured during the application operation. Another advantage is that the emulsified cosmetic can be applied to the edges of the eyelids without interference with the eyelashes. When the application portion 21 is in the above-described inclined state, the user can bring the liquid applicator 2 into abutment against the eyelashes in the manner shown in FIG. 7, so that the direction of motion of the application portion 21 (a direction along the edge of the eyelid) can be naturally brought into coincidence with the bending direction of the application portion 21 (the left-right direction). From the above points of view, it is preferable to set the angle θ between the axis "a" of the application portion 21 in its extending direction and the axis "b" of the support shaft 22 (see FIG. 5) to 80 degrees or less, and particularly 5 to 45 degrees.

When the application operation shown in FIG. 7 is performed, the application portion 21 is moved left and right along the edge of the eyelid. As described above, the application portion 21 can be bent more easily at the smallest-width section 21C in a direction on the first surface 21A than in a direction orthogonal to the first surface. Therefore, when the application portion 21 is moved left and right along the edge of the eyelid with the first surface 21A facing upward and the second surface 21B facing downward, the application portion 21 is bend in the direction indicated by reference sign D1 with the position of the smallest-width section 21C serving as a pivot axis (i.e., is pivotally rotated) as shown in FIG. 8. This bending allows the emulsified cosmetic to be more easily applied even to a narrow area, i.e., the edge of the eyelid. The bending relaxes the force when the application portion 21 comes into contact with the object area. Therefore, even when the application portion 21 comes into contact with a sensitive area such as the edge of the eyelid, a physical stimulus to the eyelid is small, so that the user can simply and safely apply the emulsified cosmetic. From the above points of view, it is preferable that, even when the application portion 21 is pressed against the edge of the eyelid and bent at an angle of, for example, about 90 degrees, the application portion 21 be bent softly to the extent that the user feels no pain. Preferably, the application portion 21 is restorable to its original shape after the application portion 21 is separated from the eyelid. This is because the application portion 21 can be easily aimed at the application position for the next application operation. For the convenience of the user of the liquid applicator 2, it is preferable that the time required to restore the original shape (the restoration rate) be not too long (not too slow), because of the ease of recognition of the touch of the application portion 21 in contact with the eyelid and the ease of control of the pressing force. The bending softness and restorableness may be determined by appropriately selecting the width and thickness of the smallest-width section 21C, the type of material forming the application portion 21, and its rubber hardness.

To more effectively cause the bending about the smallest-width section 21C as the pivot axis, it is preferable that the force for bending the application portion 21 at the smallest-width section 21C in the in-plane direction of the first surface be smaller than the force for bending the application portion 21 at the smallest-thickness section 21F in a direction orthogonal to the first surface. For a similar reason, it is preferable that the pivot axis at the smallest-width section 21C and the pivot axis of the smallest-thickness section 21F be skewed from each other. Particularly, these rotation axes cross at an angle of preferably 90° when viewed in the direction of the axis "a" of the application portion 21. In this manner, when the application portion 21 is moved left and right along the edge of the eyelid to apply the emulsified cosmetic, the application portion 21 is bent only in a direction along the moving direction (the left-right direction) and is less likely to be bent in the vertical direction. Therefore, during the application of the emulsified cosmetic to eyebrows or eyelashes, the liquid applicator 2 does not accidentally slip off the edge of the eyelid and move out of the target application area, so that the emulsified cosmetic does not adhere to the eyelid and eyeball. Accordingly, the emulsified cosmetic can be safely and easily applied to a fine area.

### [Examples]

### Examples 1 to 4 and Comparative Example 2

Water phase components shown in TABLE 1 were mixed at 25°C using the DISPER of T.K. ROBOMIX manufactured by PRIMIX Corporation to prepare a water phase. Separately, oil phase components shown in TABLE 1 were mixed at 25°C using the DISPER of T.K. ROBOMIX to prepare an oil phase. While the oil phase was mixed at 25°C using the DISPER of T.K. ROBOMIX, the previously prepared water phase was added dropwise thereto, and the oil and water phases were uniformly mixed to obtain a W/O type emulsified cosmetic for eyebrows or eyelashes.

### Comparative Example 1

Oil phase components in TABLE 1 were mixed at 25°C using the DISPER of T.K. ROBOMIX to prepare an oil-based cosmetic for eyebrows or eyelashes.

### Example 5

Oil phase components shown in TABLE 1 were used to prepare an oil phase at 25°C using the T.K. ROBOMIX. Separately, water phase components shown in TABLE 1 were mixed at 75°C using the DISPER of T.K. ROBOMIX, and the mixture was cooled to 50°C to prepare a water phase. While the water phase was mixed using the DISPER of T.K. ROBOMIX, the previously prepared oil phase was added dropwise thereto, and the water and oil phases were uniformly mixed. Then the mixture was cooled to 25°C under mixing and degassed to obtain an O/W type emulsified cosmetic for eyebrows or eyelashes.

### <Evaluation>

For each of the obtained emulsified cosmetics for eyebrows or eyelashes in Examples 1 to 5 and Comparative Example 2 and the oil-based cosmetic for eyebrows or eyelashes in Comparative Example 1, "viscosity measurement," a "hair growth and restoration effect test," an "irritation evaluation test," an "ease-of-use evaluation test," and the "ability to retain curl after application of mascara" were performed as described below. The results obtained are shown in TABLE 1.

### (Viscosity measurement)

For each of the cosmetics for eyebrows or eyelashes in the Examples and Comparative Examples, viscosity measurement was performed at 25°C using a TV-10M type viscometer manufactured by Toki Sangyo Co., Ltd. More specifically, a value displayed in the viscometer one minute after the start of rotation of the rotor of the viscometer was used as the viscosity of the cosmetic. The rotation speed and the rotor were appropriately selected according to the range of viscosity of the sample. Practically, it is preferable that the viscosity be within the range of 1 to 400 Pa·s.

### (Evaluation of hair growth and restoration effect)

For a panel of 20 subjects (12 males and 8 females), one of the cosmetics for eyebrows or eyelashes in the Examples and Comparative Examples was applied to the eyelashes of the left eye and the roots of the eyelashes in an amount of 0.015±0.009 g/eye twice a day in the morning and evening for 60 days. After 60 days, the conditions (length, thickness, and density) of the left eyelashes were compared with the conditions of the right eyelashes, and a photograph of the eyelashes of the left eye before the test was compared with a photograph after the test (the comparisons were made by the subjects themselves). The results were rated according to the following criteria using the number of subjects who evaluated that the cosmetic had a hair growth and restoration effect. Practically, an A or B rating is desirable.

For each of the emulsified cosmetics for eyebrows or eyelashes in Examples 1 to 5 and Comparative Example 2, the liquid applicator used to apply the cosmetic was the applicator shown in FIGs. 1 to 8 with bristles planted in the resin-made application portion. For the oil-based cosmetic for eyebrows or eyelashes in Comparative Example 1, a screw-like metal applicator (FIG. 9 (a partial side view of a screw-like end portion)) generally used for mascara was used. Practically, an A or B rating is desirable.

### Rating Criterion (out of a panel of 20 subjects)

A: The number of subjects who evaluated that the cosmetic had a hair growth and restoration effect was 10 or more.
B: The number of subjects who evaluated that the cosmetic had a hair growth and restoration effect was 7 to 9.
C: The number of subjects who evaluated that the cosmetic had a hair growth and restoration effect was 4 to 6.
D: The number of subjects who evaluated that the cosmetic had a hair growth and restoration effect was 3 or less.

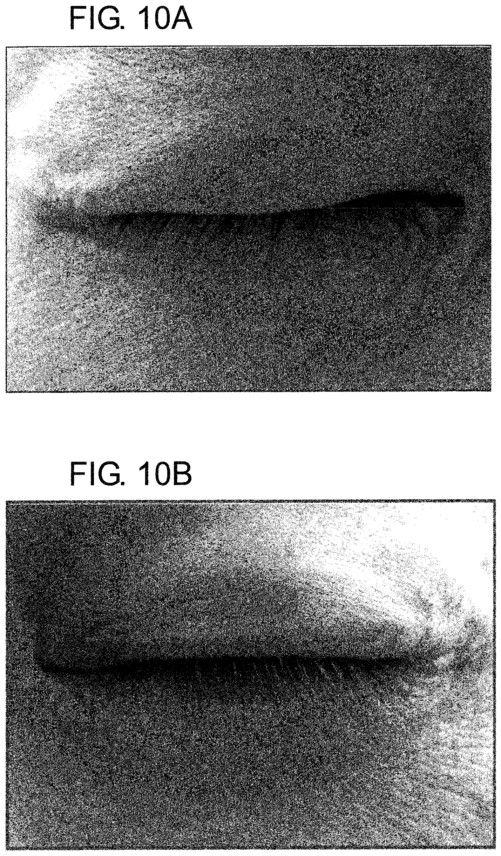

A photograph of the eyelashes of the right eye of one subject in the panel (thirties, male) on which the hair growth effect test using the emulsified cosmetic in Example 1 was not performed is shown in FIG. 10A, and a photograph of the eyelashes of the left eye on which the hair growth effect test was performed is shown in FIG. 10B.

### (Irritation evaluation)

A hearing survey on the presence of irritation during the hair growth effect evaluation was conducted. The results were rated according to the following criteria using the number of subjects who evaluated that the cosmetic had no uncomfortable irritation. Practically, an A or B rating is desirable.

### Rating Criterion (out of a panel of 20 subjects)

A: The number of subjects who did not experience uncomfortable irritation was 16 or more.
B: The number of subjects who did not experience uncomfortable irritation was 12 to 15.
C: The number of subjects who did not experience uncomfortable irritation was 8 to 11.
D: The number of subjects who did not experience uncomfortable irritation was 7 or less.

### (Ease-of-use evaluation)

A hearing survey on the ease of use during the hair growth effect evaluation (whether or not a proper amount of the cosmetic could be applied to a target area) was conducted. The results were rated according to the following criteria using the number of subjects who evaluated that the cosmetic was easy to use. Practically, an A or B rating is desirable.

### Rating Criterion (out of a panel of 20 subjects)

A: The number of subjects who evaluated that the cosmetic was easy to use was 16 or more.
B: The number of subjects who evaluated that the cosmetic was easy to use was 12 to 15.
C: The number of subjects who evaluated that the cosmetic was easy to use was 8 to 11.
D: The number of subjects who evaluated that the cosmetic was easy to use was 7 or less.

### (Evaluation of ability to retain curl after application of mascara)

A hearing survey on the ability to retain curl after application of mascara was conducted. The results were rated according to the following criteria using the number of subjects who evaluated that the ability to retain curl after application of mascara was improved. The mascara used was AUBEcouture designing mascara. Practically, an A or B rating is desirable.

### Rating Criterion (out of a panel of 20 subjects)

A: The number of subjects who evaluated that the ability to retain curl after application of the mascara was improved was 10 or more.
B: The number of subjects who evaluated that the ability to retain curl after application of the mascara was improved was 7 to 9.
C: The number of subjects who evaluated that the ability to retain curl after application of the mascara was improved was 4 to 6.
D: The number of subjects who evaluated that the ability to retain curl after application of the mascara was improved was 3 or less.

**[TABLE 1]**

| Phase | Component | Example | | | | | Comparative Example | |
|---|---|---|---|---|---|---|---|---|
| | | 1 | 2 | 3* | 4 | 5 | 1 | 2 |
| Oil phase (parts by mass) | Trans-3,4'-dimethylflavanonol | 0.10 | 0.10 | 0.01 | 0.20 | 0.10 | 0.10 | - |
| | See Synthesis Example 1 in Japanese Patent No. 3131105 | | | | | | | |
| | PEG-12 dimethicone | 0.20 | 0.20 | 0.20 | 020 | - | - | 020 |
| | SH3775M, Dow Coming Toray Co., Ltd. | | | | | | | |
| | PEG-9 polydimethylsiloxyethyl dimethicone | 0.90 | 0.90 | 0.90 | 0.90 | - | - | 0.90 |
| | KF-6028, Shin-Etsu Chemical Co., Ltd. | | | | | | | |
| | PEG-3 dimethicone | 0.90 | 0.90 | 0.90 | 0.90 | - | - | 0.90 |
| | KF-6015, Shin-Etsu Chemical Co., Ltd. | | | | | | | |
| | Liquid isoparaffin | 3.00 | 3.00 | 3.00 | 3.00 | - | - | 3.00 |
| | Parleam F, NOF Corporation | | | | | | | |
| | Isododecane | 33.00 | 35.00 | 35.09 | 60.00 | 35.00 | 90.10 | 35.10 |
| | Marukasol R, Maruzen Petrochemical Co., Ltd. | | | | | | | |
| | Decamethylcyclopentasiloxane | 2.00 | - | - | - | - | - | - |
| | KF-995, Shin-Etsu Chemical Co., Ltd. | | | | | | | |
| | Quatemium-18 hectorite | 1.60 | 1.60 | 1.60 | 1.60 | - | 7.50 | 1.60 |
| | Bentone 38VCG, Elementis Japan | | | | | | | |
| | Ethanol | 2.80 | 2.80 | 2.80 | 2.80 | - | 2.00 | 2.80 |
| | Phenoxyethanol | 0.20 | 0.20 | 0.20 | 020 | 0.10 | 0.30 | 0.20 |
| Water phase (parts by mass) | Water | 51.30 | 51.30 | 51.30 | 2820 | 55.10 | - | 51.30 |
| | Sorbitan stearate | - | - | - | - | 0.40 | - | - |
| | Rheodol SV-S10V, Kao Corporation | | | | | | | |
| | Polysorbate 60 | - | - | - | - | 0.40 | - | - |
| | Rheodol TW-S120V, Kao Corporation | | | | | | | |
| | Hydroxyethyl cellulose | - | - | - | - | 5.00 | - | - |
| | HEC Daicel SE400, Daicel Corporation | | | | | | | |
| | Dipropylene glycol | 4.00 | 4.00 | 4.00 | 2.00 | 3.50 | - | 4.00 |
| | Phenoxyethanol | - | - | - | - | 0.30 | - | - |
| | Methylparaben | - | - | - | - | 0.10 | - | - |
| Total (parts by mass) | | 100.00 | 100.00 | 100.00 | 100.00 | 100.00 | 100.00 | 100.00 |
| Type of emulsion system | | W/O | W/O | W/O | W/O | O/W | O | W/O |
| Type of applicator | | Resin + planted bristles | Resin + planted bristles | Resin + planted bristles | Resin + planted bristles | Resin + planted bristles | Metal screw | Resin + planted bristles |
| Evaluation | Viscosity (Pa·s) | 35 | 38 | 37 | 38 | 36 | 39 | 37 |
| | Hair growth and restoration effect (rating / number of subjects) | A/13 | A/14 | A/11 | A/15 | A/13 | A/14 | D/2 |
| | Irritation (rating / number of subjects) | A/20 | A/20 | A/20 | A/16 | A/20 | D/1 | A/20 |
| | Ease of use (rating / number of subjects) | A/18 | A/18 | A/18 | A/18 | A/17 | B/15 | A/18 |
| | Ability to retain curl after application of mascara (rating / number of subjects) | A/13 | A/15 | A/11 | A/16 | A/14 | A/14 | D/2 |

| | | | | | | | | |
|---|---|---|---|---|---|---|---|---|
| * Example not falling within the scope of the invention as claimed | | | | | | | | |

As shown in TABLE 1, in each of the emulsified cosmetics for eyebrows or eyelashes in Examples 1 to 5, a solution prepared by dissolving a predetermined amount of the flavanonol derivative of the formula (1) in a predetermined amount of a specific volatile oil forms the oil phase of the emulsion system. Therefore, the emulsified cosmetic has a favorable growth and restoration effect on eyelashes and eyebrows. Since bounce and resilience can be imparted to the eyelashes, the ability to retain curl after application of mascara is improved. In addition, irritation to the eyes is small, and the ease of use of the cosmetic is high because the cosmetic is prepared so as to have a viscosity suitable for use. Particularly, as for the hair growth and restoration effect, the number, length, and thickness of eyelashes were obviously larger for the left eye to which the emulsified cosmetic in Example 1 was applied (FIG. 10B) than for the right eye to which the emulsified cosmetic was not applied (FIG. 10A).

Comparative Example 1 is an oil-based cosmetic for eyebrows or eyelashes, and irritation due to the organic solvent was strong. The cosmetic for eyebrows or eyelashes in Comparative Example 2 does not contain the flavanonol derivative of the formula (1). Therefore, the growth and restoration effect on eyelashes and eyebrows was not obtained. In addition, the ability to retain curl after application of mascara was lower than that in the Examples.

### Example 6 and Comparative Examples 3 to 5

Water phase components shown in TABLE 2 were mixed at 25°C using the DISPER of T.K. ROBOMIX manufactured by PRIMIX Corporation to prepare a water phase. Separately, oil phase components shown in TABLE 2 were mixed at 25°C using the DISPER of T.K. ROBOMIX to prepare an oil phase. While the oil phase was mixed at 25°C using the DISPER of T.K. ROBOMIX, the previously prepared water phase was added dropwise thereto, and the oil and water phases were uniformly mixed to obtain a W/O type emulsified cosmetic for eyebrows or eyelashes.

### <Evaluation>

For each of the obtained emulsified cosmetics for eyebrows or eyelashes in Example 6 and Comparative Examples 3 to 5, "viscosity measurement," "irritation evaluation," "ease-of-use evaluation," "evaluation of ability not to cause dry skin around eyes," and "evaluation of elasticity of skin around eyes" were performed as described below. The results obtained are shown in TABLE 2.

The viscosity of the emulsified cosmetic for eyebrows or eyelashes was measured using a B-type viscometer (TVB-10M, Toki Sangyo Co., Ltd) under the condition of 25°C with a rotor M3 rotating at a rotation speed of 1.5 r/min when the viscosity was 80 Pa·s or less. When the viscosity exceeded 80Pa·s, the measurement was performed with the rotor M3 rotating at a rotation speed of 0.3 r/min.

### (Irritation evaluation)

For ten expert panelists, the emulsified cosmetic for eyebrows or eyelashes was applied to the eyebrows and the roots of the eyelashes in the morning, and the number of panelists who evaluated that no uncomfortable irritation was experienced even in the evening was counted.

### (Ease-of-use evaluation)

The ease of use of the emulsified cosmetic for eyebrows or eyelashes when it was applied to the eyebrows and the roots of the eyelashes in the morning (whether or not a proper amount of the emulsified cosmetic could be applied to the target areas) was evaluated by ten expert panelists. The number of panelists who evaluated that the emulsified cosmetic was easy to use was counted.

### (Evaluation of ability not to cause dry skin around eyes)

For ten expert panelists, the emulsified cosmetic

For ten expert panelists, the emulsified cosmetic for eyebrows or eyelashes was applied to the eyebrows and the roots of the eyelashes in the morning, and the number of panelists who evaluated that the skin around the eyes was soft even in the evening was counted.
for eyebrows or eyelashes was applied to the eyebrows and the roots of the eyelashes in the morning, and the number of panelists who evaluated that no dry skin around the eyes was experienced even in the evening was counted.

### (Evaluation of elasticity of skin around eyes)

**[TABLE 2]**

| Phase | Component | Example | Comparative Example | | |
|---|---|---|---|---|---|
| | | 6 | 3 | 4 | 5 |
| Oil phase (parts by mass) | (A) Trans-3,4'-dimethylftavanonol | 0.10 | 0.10 | 0.10 | - |
| | See Synthesis Example 1 in Japanese Patent No. 3131105 | | | | |
| | PEG-12 dimethicone | 0.20 | 0.20 | 0.20 | 0.20 |
| | SH3775M, Dow Coming Toray Co., Ltd. | | | | |
| | PEG-9 polydimethylsiloxyethyl dimethicone | Ltd. 0.90 | 0.90 | 0.90 | 0.90 |
| | KF-6028, Shin-Etsu Chemical Co., | | | | |
| | PEG-3 dimethicone | 0.90 | 0.90 | 0.90 | 0.90 |
| | KF-6015, Shin-Etsu Chemical Co., Ltd. | | | | |
| | Liquid isoparaffin | 3.00 | 38.0 | - | 3.00 |
| | Parleam F, NOF Corporation | | | | |
| | Isononyl isononanoate | - | - | 38.0 | - |
| | (B1) Isododecane | 33.00 | - | - | 33.0 |
| | Marukasol R, Maruzen Petrochemical Co., Ltd. | | | | |
| | (B2) Decamethylcyclopentasiloxane | 2.00 | - | - | 2.00 |
| | KF-995, Shin-Etsu Chemical Co., Ltd. | | | | |
| | Quatemium-18 hectorite | 1.60 | 1.60 | 1.60 | 1.60 |
| | Bentone 38VCG, Elementis Japan | | | | |
| | Ethanol | 2.80 | 2.80 | 2.80 | 2.80 |
| | Phenoxyethanol | 0.20 | 0.20 | 0.20 | 0.20 |
| Water phase (parts by mass) | Water | 55.30 | 55.30 | 55.30 | 55.40 |
| Total (parts by mass) | | 100.00 | 100.00 | 100.00 | 100.00 |
| Type of emulsion system | | W/O | W/O | W/O | W/O |
| Type of applicator | | Resin + planted bristles | Resin + planted bristles | Resin + planted bristles | Resin + planted bristles |
| [mass of volatile oils]/[mass of flavanonol] = {(B1) + (B2)}/(A) | | 350 | - | - | - |
| Evaluation | Viscosity (Pa·s) | 34 | 37 | 31 | 35 |
| | Irritation (Number of panelists) | 10 | 10 | 10 | 10 |
| | Ease of use (Number of panelists) | 9 | 4 | 5 | 9 |
| | Ability not to cause dry skin around eyes (Number of panelists) | 10 | 3 | 2 | 3 |
| | Elasticity of skin (Number of panelists) | 9 | 2 | 2 | 2 |

As shown in TABLE 2, in the emulsified cosmetic for eyebrows or eyelashes in Example 6, a solution prepared by dissolving a predetermined amount of the flavanonol derivative of the formula (1) in predetermined amounts of specific volatile oils forms the oil phase of the emulsion system. Therefore, the emulsified cosmetic did not cause irritation, was easy to use, did not cause dryness around the eyes, and allowed the skin around the eyes to be elastic.

However, in the emulsified cosmetics for eyebrows or eyelashes in Comparative Examples 3 and 4, the predetermined volatile oils were not used, and a nonvolatile oil other than the volatile oils (liquid isoparaffin or isononyl isononanoate (ester oil)) was used. Therefore, the ease of use was low, the skin around the eyes was more likely to be dry and was less elastic. In the emulsified cosmetic for eyebrows or eyelashes in Comparative Example 5, the specific flavanonol derivative was not used. Therefore, the skin around the eyes was more likely to be dry, and the skin was less elastic.

### Examples 7 to 12 and Comparative Examples 6 and 7

Water phase components shown in TABLE 3 were mixed at 25°C using the DISPER of T.K. ROBOMIX manufactured by PRIMIX Corporation to prepare a water phase. Separately, oil phase components shown in TABLE 3 were mixed at 25°C using the DISPER of T.K. ROBOMIX to prepare an oil phase. While the oil phase was mixed at 25°C using the DISPER of T.K. ROBOMIX, the previously prepared water phase was added dropwise thereto, and the oil and water phases were uniformly mixed to obtain a W/O type emulsified cosmetic for eyebrows or eyelashes.

### <Evaluation>

For each of the obtained emulsified cosmetics for eyebrows or eyelashes in Examples 7 to 12 and Comparative Examples 6 and 7, "viscosity measurement," "irritation evaluation," "ease-of-use evaluation," "evaluation of ability not to cause dry skin around eyes," and "evaluation of elasticity of skin around eyes" were performed as in Example 6. The results obtained are shown in TABLE 3.

**[TABLE 3]**

| Phase | Component | Comparative Example | Example | | | | | Comparative Example | Example | |
|---|---|---|---|---|---|---|---|---|---|---|
| | | 6 | 7 | 8 | (6) | 9 | 10 | 7 | 11 | 12 |
| | (A) Trans-3,4'-dimethylflavanonol | 0.10 | 0.10 | 0.10 | 0.10 | 0.10 | 0.10 | 0.10 | 0.05 | 0.20 |
| | See Synthesis Example 1 in Japanese Patent No. 3131105 | | | | | | | | | |
| | PEG-12 dimethicone | 0.20 | 0.20 | 0.20 | 0.20 | 0.20 | 0.20 | 0.20 | 0.20 | 0.20 |
| | SH3775M, Dow Coming Toray Co., Ltd. | | | | | | | | | |
| | PEG-9 polydimethylsiloxyethyl dimethicone | 0.90 | 0.90 | 0.90 | 0.90 | 0.90 | 0.90 | 0.90 | 0.90 | 0.90 |
| | KF-6028, Shin-Etsu Chemical Co., Ltd. | | | | | | | | | |
| | PEG-3 dimethicone | 0.90 | 0.90 | 0.90 | 0.90 | 0.90 | 0.90 | 0.90 | 0.90 | 0.90 |
| | KF-6015, Shin-Etsu Chemical Co., Ltd. | | | | | | | | | |
| oil phase | Liquid isoparaffin | 3.00 | 3.00 | 3.00 | 3.00 | 3.00 | 3.00 | 3.00 | 3.00 | 3.00 |
| (parts by mass) | Parleam F, NOF Corporation | | | | | | | | | |
| | Isononyl isononanoate | - | - | - | - | - | - | - | - | - |
| | (B1) Isododecane | 0.50 | 8.00 | 18.00 | 33.00 | 43.00 | 58.00 | 78.00 | 33.00 | 33.00 |
| | Marukasol R, Maruzen Petrochemical Co., Ltd. | | | | | | | | | |
| | (B2) Decamethylcyclopentasiloxane | 2.00 | 2.00 | 2.00 | 2.00 | 2.00 | 2.00 | 2.00 | 2.00 | 2.00 |
| | KF-995, Shin-Etsu Chemical Co., Ltd. | | | | | | | | | |
| | Quatemium-18 hectorite | 1.60 | 1.60 | 1.60 | 1.60 | 1.60 | 1.60 | 1.60 | 1.60 | 1.60 |
| | Bentone 38VCG, Elementis Japan | | | | | | | | | |
| | Ethanol | 2.80 | 2.80 | 2.80 | 2.80 | 2.80 | 2.80 | 2.80 | 2.80 | 2.80 |
| | Phenoxyethanol | 0.20 | 0.20 | 0.20 | 0.20 | 0.20 | 0.20 | 0.20 | 0.20 | 0.20 |
| Water phase (parts by mass) | Water | 87.80 | 80.30 | 70.30 | 55.30 | 45.30 | 30.30 | 10.30 | 55.35 | 55.20 |
| Total (parts by mass) | | 100.00 | 100.00 | 100.00 | 100.00 | 100.00 | 100.00 | 100.00 | 100.00 | 100.00 |
| Type of emulsion system | | W/O | W/O | W/O | W/O | W/O | W/O | W/O | W/O | W/O |
| Type of applicator | | Resin + planted bristles | Resin + planted bristles | Resin + planted bristles | Resin + planted bristles | Resin + planted bristles | Resin + planted bristles | Resin + planted bristles | Resin + planted bristles | Resin + planted bristles |
| mass of volatile oils]/[mass of flavanonol] = {(B1) + (B2)}/(A) | | 25 | 100 | 200 | 350 | 450 | 600 | 800 | 700 | 175 |
| Evaluation | Viscosity (Pa·s) | 145 | 128 | 38 | 34 | 33 | 31 | 27 | 34 | 35 |
| | Irritation (Number of panelists) | 10 | 10 | 10 | 10 | 9 | 8 | 2 | 10 | 10 |
| | Ease of use (Number of panelists) | 8 | 8 | 9 | 9 | 9 | 9 | 9 | 9 | 9 |
| | Ability not to cause dry skin around eyes (Number of panelists) | 4 | 8 | 10 | 10 | 10 | 8 | 4 | 7 | 8 |
| | Elasticity of skin (Number of panelists) | 2 | 7 | 9 | 9 | 9 | 7 | 2 | 6 | 7 |

As shown in TABLE 3, in each of the emulsified cosmetics for eyebrows or eyelashes in Examples 7 to 12, a solution prepared by dissolving a predetermined amount of the flavanonol derivative of the formula (1) in predetermined amounts of specific volatile oils forms the oil phase of the emulsion system. Therefore, each emulsified cosmetic was not viscous, caused no irritation, was easy to use, did not cause dryness around the eyes, and allowed the skin to be highly elastic. Particularly, as can be seen from the results for the emulsified cosmetics for eyebrows or eyelashes in Examples 8 and 9, particularly preferred results are obtained when the contained amount of the specific volatile oils is 20.00 to 35.00% by mass. As can be seen from the results for Examples 11 and 12, preferred results are obtained when the contained amount of the specific flavanonol derivative is in the range of 0.05 to 0.20% by mass.

As can be seen from the results for Comparative Examples 6 and 7, if the contained amount of the specific volatile oils is too small or too large, dryness around the eyes is more likely to occur, and the elasticity of the skin around the eyes is also reduced.

### Industrial Applicability

In the emulsified cosmetic for eyebrows or eyelashes of the present invention, a solution prepared by dissolving a predetermined amount of the flavanonol derivative of the formula (1) in a predetermined amount of a specific volatile oil forms the oil phase of the emulsion system. Therefore, the emulsified cosmetic for eyebrows or eyelashes of the present invention has a viscosity suitable for use and shows a favorable growth and restoration effect on eyelashes or eyebrows. The emulsified cosmetic of the present invention also shows a favorable bounce and resilience-imparting effect on eyelashes, causes less irritation to the eyes, and provides high usability. In addition, the emulsified cosmetic does not cause dryness around the eyes and allows the skin to be highly elastic. Therefore, the emulsified cosmetic is useful as an emulsified cosmetic for growing and restoring eyebrows or eyelashes.

According to the above-described embodiments, the present invention further discloses the following cosmetics.
<1> An emulsified cosmetic for eyebrows or eyelashes, containing 0.05 % to 0.2 % by mass of a flavanonol derivative represented by the following formula (1) and 5 to 65% by mass of a volatile oil selected from volatile hydrocarbon oils and volatile silicone oils: 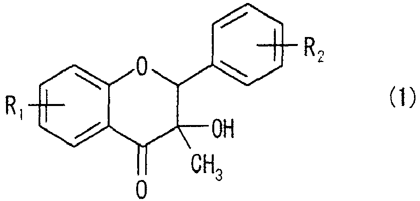 in the formula, R₁ and R₂ are each independently a hydrogen atom or a methyl group, wherein a mass ratio of the volatile oil to the flavanonol derivative being (parts by mass of the volatile oil)/(parts by mass of the flavanonol derivative) is 50 to 700.
<2> The emulsified cosmetic for eyebrows or eyelashes according to <1>, wherein the contained amount of the volatile oil is preferably 10% by mass or more, more preferably 15% by mass or more, and still more preferably 20% by mass or more and is preferably 60% by mass or less, more preferably 55% by mass or less, and still more preferably 45% by mass or less.
<3> The emulsified cosmetic for eyebrows or eyelashes according to <1> or <2>, forming a W/O type emulsion system.
<4> The emulsified cosmetic for eyebrows or eyelashes according to any of <1> to <3>, wherein the amount of a water phase contained in the W/O type emulsion system is preferably 35% by mass or more, more preferably 40% by mass or more, and still more preferably 45% by mass or more and is preferably 95% by mass or less, more preferably 85% by mass or less, and still more preferably 75% by mass or less.
<5> The emulsified cosmetic for eyebrows or eyelashes according to any of <1> to <4>, wherein the flavanonol derivative is preferably trans-3,4'-dimethylflavanonol or trans-3,7-dimethylflavanonol, and more preferably trans-3,4'-dimethylflavanonol.
<6> The emulsified cosmetic for eyebrows or eyelashes according to any of <1> to <5>, wherein a flash point of the volatile oil is preferably 35 to 87°C.
<7> The emulsified cosmetic for eyebrows or eyelashes according to any of <1> to <6>, wherein the volatile oil is preferably a volatile hydrocarbon oil, more preferably an isoparaffinic hydrocarbon oil having 8 to 18 carbon atoms, and still more preferably isododecane.
<8> The emulsified cosmetic for eyebrows or eyelashes according to any of <1> to <7>, wherein the contained amount of the flavanonol derivative is 0.08% by mass or more and is 0.12% by mass or less.
<9> The emulsified cosmetic for eyebrows or eyelashes according to any of <1> to <8>, wherein the mass ratio of the volatile oil to the flavanonol derivative being (parts by mass of the volatile oil)/(parts by mass of the flavanonol derivative) is 100 parts by mass or more, and preferably 200 parts by mass and is 600 parts by mass or less, and preferably 450 parts by mass or less.
<10> The emulsified cosmetic for eyebrows or eyelashes according to any of <1> to <9>, having a viscosity at 25°C of preferably 1 Pa·s or more, more preferably 5 Pa·s or more, and still more preferably 10 Pa·s or more and of preferably 400 Pa·s or less, more preferably 300 Pa·s or less, and still more preferably 200 Pa·s or less.
<11> The emulsified cosmetic for eyebrows or eyelashes according to any of <1> to <10>, further containing an organic-modified clay mineral obtained by substituting an interlayer sodium ion in a laminar clay mineral such as bentonite, magnesium aluminum silicate, Laponite, or hectorite with a quaternary ammonium ion.
<12> The emulsified cosmetic for eyebrows or eyelashes according to any of <1> to <10>, wherein
   the organic-modified viscous mineral is at least one selected from the group consisting of dimethyl alkyl ammonium hectorite, benzyl dimethyl stearyl ammonium hectorite, and ammonium magnesium silicate treated with distearyl dimethyl ammonium chloride.
<13> The emulsified cosmetic for eyebrows or eyelashes according to any of <1> to <12>, wherein the contained amount of the organic-modified clay mineral is preferably 0.1% by mass or more, more preferably 0.2% by mass or more, still more preferably 0.3% by mass or more, and yet more preferably 0.5% by mass and is preferably 10% by mass or less, more preferably 7% by mass or less, still more preferably 5% by mass or less, and yet more preferably 4% by mass or less.
<14> The emulsified cosmetic for eyebrows or eyelashes according to any of <1> to <13>, further containing a silicone-based emulsifier in an amount of preferably 0.1% by mass or more, more preferably 0.5% by mass or more, and still more preferably 1% by mass or more and of 5% by mass or less, more preferably 4% by mass or less, and still more preferably 3% by mass or less.
<15> A packaged emulsified cosmetic product for eyebrows or eyelashes which is used to grow and restore the eyebrows or the eyelashes, the packaged emulsified cosmetic product comprising: the emulsified cosmetic for eyebrows or eyelashes according to any of <1> to <14>; a container that contains the emulsified cosmetic for eyebrows or eyelashes; a lid capable of sealing the container; and a liquid applicator including a support shaft attached to the lid so as to be inserted into the container and an application portion attached to an end of the support shaft.
<16> An emulsified growth and restoration agent for eyebrows or eyelashes, containing: 0.05 % to 0.2 % by mass of a flavanonol derivative represented by the formula (1); 5 to 65% by mass of a volatile oil selected from volatile hydrocarbon oils and volatile silicone oils; and water, wherein the flavanonol derivative is dissolved in the volatile oil in an oil phase.
<17> Use of an emulsified cosmetic for eyebrows or eyelashes according to any of <1> to <15> or a growth and restoration agent for eyebrows or eyelashes according to <16>, wherein the emulsified cosmetic or the growth and restoration agent is applied to eyebrows or eyelashes.
<18> Use of an emulsified cosmetic for eyebrows or eyelashes according to any of <1> to <15> to produce an emulsified cosmetic for eyebrows or eyelashes.
<19> A method of use of an emulsified cosmetic for eyebrows or eyelashes according to any of <1> to <15>, comprising applying the emulsified cosmetic for eyebrows or eyelashes to skin around roots of the eyebrows or the eyelashes once a day in order to promote growth and restoration of the eyebrows or the eyelashes.
<20> The method of use according to <19>, wherein the amount of the emulsified cosmetic per application for one eye is preferably 0.002 g or more, more preferably 0.003 g or more, and still more preferably 0.005 g or more and is preferably 0.04 g or less, more preferably 0.03 g or less, and still more preferably 0.025 g or less. Reference Signs List

- 1: "Packaged emulsified cosmetic product"
- 2: Liquid applicator
- 21: Application portion
- 21A: First surface
- 21B: Second surface
- 21C: Smallest-width section
- 21D: Forward end
- 21E: Large-width section
- 21F: Smallest-thickness section
- 21G: Largest-thickness section
- 22: Support shaft
- 23: Lid
- 24: Joint portion
- 3: Container
- 31: Scraping valve
- 31A: Scraping opening
- 32: Threaded portion
- 33: Opening
- 4: Emulsified cosmetic for eyebrows or eyelashes

## Claims

1. An emulsified cosmetic for eyebrows or eyelashes, comprising: 0.05 to 0.2% by mass of a flavanonol derivative represented by the following formula (1) 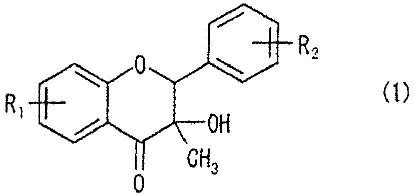 in the formula, R₁ and R₂ are each independently a hydrogen atom or a methyl group; and 5 to 65% by mass of a volatile oil selected from volatile hydrocarbon oils and volatile silicone oils, wherein a mass ratio of the volatile oil to the flavanonol derivative being (parts by mass of the volatile oil)/(parts by mass of the flavanonol derivative) is 50 to 700.

2. The emulsified cosmetic for eyebrows or eyelashes according to claim 1, forming a W/O type emulsion system.

3. The emulsified cosmetic for eyebrows or eyelashes according to claim 1 or 2, wherein the flavanonol derivative is trans-3,4'-dimethylflavanonol or trans-3,7-dimethylflavanonol.

4. The emulsified cosmetic for eyebrows or eyelashes according to any one of claims 1 to 3, wherein the volatile oil has a flash point of 35 to 87°C.

5. The emulsified cosmetic for eyebrows or eyelashes according to any one of claims 1 to 4, wherein the volatile oil is an isoparaffinic hydrocarbon oil having 8 to 18 carbon atoms.

6. The emulsified cosmetic for eyebrows or eyelashes according to any one of claims 1 to 5, wherein a contained amount of the volatile oil is 10 to 60% by mass.

7. The emulsified cosmetic for eyebrows or eyelashes according to any one of claims 1 to 6, having a viscosity at 25°C of 1 to 400 Pa·s.

8. The emulsified cosmetic for eyebrows or eyelashes according to any one of claims 1 to 7, being a W/O type emulsion system, and wherein an amount of a water phase contained is 40 to 85% by mass.

9. The emulsified cosmetic for eyebrows or eyelashes according to any one of claims 1 to 8, further comprising an organic-modified clay mineral in an amount of 0.1 to 10% by mass.

10. The emulsified cosmetic for eyebrows or eyelashes according to any one of claims 1 to 9, further comprising a silicone-based emulsifier in an amount of 0.1 to 5% by mass.

11. A packaged emulsified cosmetic product for eyebrows or eyelashes which is used to grow and restore the eyebrows or the eyelashes, the packaged emulsified cosmetic product comprising: the emulsified cosmetic for eyebrows or eyelashes according to any one of claims 1 to 10; a container that contains the emulsified cosmetic for eyebrows or eyelashes; a lid capable of sealing the container; and a liquid applicator including a support shaft attached to the lid so as to be inserted into the container and an application portion attached to an end of the support shaft.

12. Use once a day of the emulsified cosmetic according to any of the claims 1 to 11, as a cosmetic for eyebrows or eyelashes.

13. The use according to claim 12, for promoting growth and restoration of the eyebrows or the eyelashes.

## Patentansprüche

1. Emulgiertes Kosmetikum für Augenbrauen oder Wimpern, umfassend: 0,05 bis 0,2 Massen-% eines Flavanonolderivats, dargestellt durch die folgende Formel (1) : 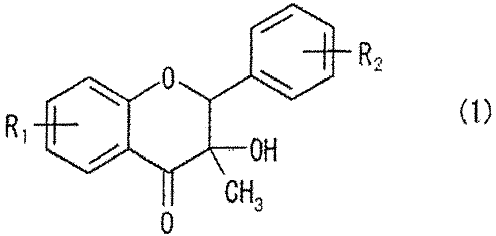 wobei in der Formel R₁ und R₂ jeweils unabhängig voneinander ein Wasserstoffatom oder eine Methylgruppe sind; und 5 bis 65 Massen-% eines flüchtigen Öls, das aus flüchtigen Kohlenwasserstoffölen und flüchtigen Silikonölen ausgewählt ist, wobei ein Massenverhältnis des flüchtigen Öls zu dem Flavanonolderivat (Masseteile des flüchtigen Öls)/(Masseteile des Flavanonolderivats) 50 bis 700 ist.

2. Emulgiertes Kosmetikum für Augenbrauen oder Wimpern gemäß Anspruch 1, das ein Emulsionssystem vom Wasser/Öl (W/O)-Typ bildet.

3. Emulgiertes Kosmetikum für Augenbrauen oder Wimpern gemäß Anspruch 1 oder 2, wobei das Flavanonolderivat trans-3,4'-Dimethylflavanonol oder trans-3,7-Dimethylflavanonol ist.

4. Emulgiertes Kosmetikum für Augenbrauen oder Wimpern gemäß einem der Ansprüche 1 bis 3, wobei das flüchtige Öl einen Flammpunkt von 35 bis 87°C aufweist.

5. Emulgiertes Kosmetikum für Augenbrauen oder Wimpern gemäß einem der Ansprüche 1 bis 4, wobei das flüchtige Öl ein isoparaffinisches Kohlenwasserstofföl mit 8 bis 18 Kohlenstoffatomen ist.

6. Emulgiertes Kosmetikum für Augenbrauen oder Wimpern gemäß einem der Ansprüche 1 bis 5, wobei eine enthaltene Menge an dem flüchtigen Öl 10 bis 60 Massen-% beträgt.

7. Emulgiertes Kosmetikum für Augenbrauen oder Wimpern gemäß einem der Ansprüche 1 bis 6, das eine Viskosität bei 25°C von 1 bis 400 Pa·s aufweist.

8. Emulgiertes Kosmetikum für Augenbrauen oder Wimpern gemäß einem der Ansprüche 1 bis 7, das ein Emulsionssystem vom W/O-Typ ist und wobei eine Menge an enthaltener Wasserphase 40 bis 85 Massen-% beträgt.

9. Emulgiertes Kosmetikum für Augenbrauen oder Wimpern gemäß einem der Ansprüche 1 bis 8, zusätzlich umfassend ein organisch modifiziertes Tonmineral in einer Menge von 0,1 bis 10 Massen-%.

10. Emulgiertes Kosmetikum für Augenbrauen oder Wimpern gemäß einem der Ansprüche 1 bis 9, zusätzlich umfassend einen Emulgator auf Silikonbasis in einer Menge von 0,1 bis 5 Massen-%.

11. Verpacktes emulgiertes Kosmetikprodukt für Augenbrauen oder Wimpern, das zum Wachsen und Wiederherstellen der Augenbrauen oder der Wimpern verwendet wird, wobei das verpackte emulgierte Kosmetikprodukt umfasst: das emulgierte Kosmetikum für Augenbrauen oder Wimpern gemäß einem der Ansprüche 1 bis 10; einen Behälter, der das emulgierte Kosmetikum für Augenbrauen oder Wimpern enthält; einen Deckel, der in der Lage ist, den Behälter zu verschließen; und einen Flüssigkeitsapplikator, der einen Trageschaft, der an dem Deckel angebracht ist, um in den Behälter eingeführt zu werden, und einen Aufbringungsteil, der an einem Ende des Trageschafts angebracht ist, einschließt.

12. Verwendung einmal täglich des emulgierten Kosmetikums gemäß einem der Ansprüche 1 bis 11 als Kosmetikum für Augenbrauen oder Wimpern.

13. Verwendung gemäß Anspruch 12 zur Förderung des Wachstums und der Wiederherstellung der Augenbrauen oder der Wimpern.

## Revendications

1. Émulsion cosmétique pour sourcils ou cils, comprenant : 0,05 à 0,2 % en masse d'un dérivé de flavanonol représenté par la formule (1) suivante 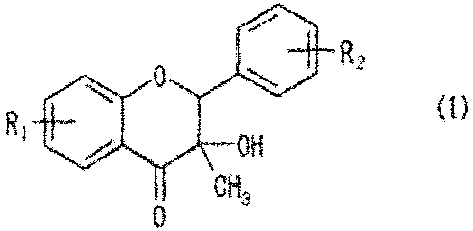 dans la formule, R₁ et R₂ sont chacun indépendamment un atome d'hydrogène ou un groupe méthyle ; et 5 à 65 % en masse d'une huile volatile sont sélectionnés parmi des huiles volatiles hydrocarbures et des huiles volatiles de silicone, dans laquelle un rapport en masse de l'huile volatile au dérivé de flavanonol (en parties en masse de l'huile volatile) / (parties en masse de dérivé de flavanonol) est de 50 à 700.

2. Émulsion cosmétique pour sourcils ou cils selon la revendication 1, formant un système d'émulsion de type E/H.

3. Émulsion cosmétique pour sourcils ou cils selon la revendication 1 ou 2, dans laquelle le dérivé de flavanonol est un trans-3,4'-diméthylflavanonol ou un trans-3,7-diméthylflavanonol.

4. Émulsion cosmétique pour sourcils ou cils selon l'une quelconque des revendications 1 à 3, dans laquelle l'huile volatile a un point d'éclair de 35 à 87 °C.

5. Émulsion cosmétique pour sourcils ou cils selon l'une quelconque des revendications 1 à 4, dans laquelle l'huile volatile est une huile hydrocarbure isoparaffinique ayant 8 à 18 atomes de carbone.

6. Émulsion cosmétique pour sourcils ou cils selon l'une quelconque des revendications 1 à 5, dans laquelle une quantité contenue de l'huile volatile est de 10 à 60 % en masse.

7. Émulsion cosmétique pour sourcils ou cils selon l'une quelconque des revendications 1 à 6, ayant une viscosité à 25 °C de 1 à 400 Pa.s.

8. Émulsion cosmétique pour sourcils ou cils selon l'une quelconque des revendications 1 à 7, qui est un système d'émulsion de type E/H, et dans laquelle une quantité de phase aqueuse contenue est de 40 à 85 % en masse.

9. Émulsion cosmétique pour sourcils ou cils selon l'une quelconque des revendications 1 à 8, comprenant en outre un minéral argileux modifié par un composant organique en une quantité de 0,1 à 10 % en masse.

10. Émulsion cosmétique pour sourcils ou cils selon l'une quelconque des revendications 1 à 9, comprenant en outre un émulsifiant à base de silicone en une quantité de 0,1 à 5 % en masse.

11. Produit d'émulsion cosmétique conditionnée pour sourcils ou cils qui est utilisé pour développer et restaurer les sourcils ou les cils, le produit d'émulsion cosmétique conditionné comprenant : l'émulsion cosmétique pour sourcils ou cils selon l'une quelconque des revendications 1 à 10 ; un contenant qui contient l'émulsion cosmétique pour sourcils ou cils ; un capuchon capable de fermer hermétiquement le contenant ; et un applicateur de liquide incluant une tige de support fixée au capuchon de façon à être inséré dans le contenant et une partie d'application fixée à une extrémité de la tige de support.

12. Utilisation une fois par jour de l'émulsion cosmétique selon l'une quelconque des revendications 1 à 11, comme cosmétique pour sourcils ou cils.

13. Utilisation selon la revendication 12, pour favoriser la croissance et la restauration des sourcils ou des cils.
